# EUROPEAN PATENT APPLICATION

(11) **EP 4 729 634 A1**
(43) Date of publication of application: **22.04.2026**
(21) Application number: 24826253.7
(22) Date of filing: 19.06.2024
(51) Int. Cl.: C12Q 1/6883

(54) **COMPOSITION AND KIT FOR DIAGNOSIS OR PROGNOSIS PREDICTION OF PACHYCHOROID SPECTRUM DISEASE, AND METHOD FOR PROVIDING INFORMATION FOR DIAGNOSIS OR PROGNOSIS PREDICTION**

(30) Priority: 19.06.2023 KR 20230078226
(71) Applicant: The Asan Foundation, Seoul 05505 (KR); UNIVERSITY OF ULSAN FOUNDATION FOR INDUSTRY COOPERATION, Nam-gu Ulsan 44610 (KR)
(72) Inventor: LEE, Junyeop, Seoul 05555 (KR); KIM, Soo Jin, Daegu 42471 (KR); YANG, Jee Myung, Seoul 05505 (KR)
(74) Representative: Beck Greener LLP
(86) International application number: PCT/KR2024/008483
(87) International publication number: WO 2024/262938

(57) **Abstract**

The present invention relates to a composition and a kit for diagnosis or prognosis prediction of pachychoroid spectrum disease, and a method for predicting therapeutic responsiveness to pachychoroid spectrum disease.

## Description

### [Technical Field]

### CROSS-REFERENCE TO RELATED APPLICATION

This application claims the benefit of priority to Korean Patent Application No. 10-2023-0078226, filed in the Korean Intellectual Property Office on June 19, 2023, the entire contents of which are incorporated herein by reference.

The present disclosure relates to a technology regarding a composition and a kit for diagnosis or prognosis prediction of pachychoroid spectrum disease, and a method for providing information for diagnosis or prognosis prediction of pachychoroid spectrum disease.

### [Background Art]

Pachychoroid spectrum disease refers to a condition in which choroidal thickening is accompanied by changes in the retinal pigment epithelium, causing retinal abnormalities, and represents a comprehensive group of disorders including central serous chorioretinopathy, polypoidal choroidal vasculopathy, pachychoroid pigment epitheliopathy, and peripapillary pachychoroid. It is thought that when the diameter of Haller's layer choroidal vessels increases in pachychoroid spectrum diseases, the relative choroidal pressure rises, which may secondarily cause functional deterioration of the retinal pigment epithelium.

By way of example, among them, central serous chorioretinopathy (CSC) is the fourth most common retinal disease, affecting 10 men and 2 women per 100,000 individuals annually. The main pathogenesis of central serous chorioretinopathy is choroidal hyperpermeability and leakage of fluid into the subretinal space through the retinal pigment epithelium (RPE), which causes significant central vision loss. Central serous chorioretinopathy is characterized by choroidal vascular pathology (e.g., choroidal thickening (pachyvessels) and choroidal venous congestion), and choroidal endothelial cells (CECs) play an important role in the progression of the disease. CECs are fenestrated, and their function is essential for regulating endothelial transport and pressure in the choroidal vasculature in central serous chorioretinopathy.

Most patients recover spontaneously within three months after onset, but the condition may recur or persist for more than three months. In such cases, active treatments such as photodynamic therapy, laser photocoagulation, mineralocorticoid receptor antagonists, and intravitreal anti-vascular endothelial growth factor (anti-VEGF) injections are required.

In patients with pachychoroid spectrum disease, anti-VEGF therapy is used to reduce the hyperpermeability of choroidal endothelial cells. Despite the lack of definitive clinical trials, anti-VEGF agents such as bevacizumab, ranibizumab, and aflibercept have been reported to exert beneficial effects in patients with pachychoroid spectrum disease by reducing subretinal fluid levels. Among pachychoroid spectrum diseases, central serous chorioretinopathy and age-related macular degeneration (AMD) are distinct disorders; however, both share several common features, such as choroidal hyperpermeability and neovascular formation. Furthermore, patients with pachychoroid spectrum disease may progress to exudative AMD. However, the pathogenesis and therapeutic targets of pachychoroid spectrum disease are not well known. Therefore, information is limited regarding specific individuals with pachychoroid spectrum disease who, like AMD patients, do not benefit from anti-VEGF therapy and exhibit suboptimal treatment responses. Therefore, distinguishing this subset of patients is highly practical for designing therapeutic strategies.

Meanwhile, exosomes are nanosized vesicles (50-175 nm in diameter) including a lipid bilayer that contain proteins and nucleic acids, which can be utilized as biomarkers. Exosomes are secreted into the extracellular environment by many types of cells and can be detected in various body fluids, such as serum, urine, saliva, and aqueous humor (AH). The main components of exosomes are proteins and lipids, and they contain RNAs such as messenger RNA (mRNA) and small RNA, which are transported to nearby target cells. Because exosomes are partially involved in intercellular communication through the transfer of exosomal RNA between cells, they have recently emerged as key indicators of human diseases.

Furthermore, microRNAs (miRNAs) are single short strands (19-22 nt) that regulate post-transcriptional processes through degradation or suppression of their targets. Gene regulation during miRNA generation, proliferation, and differentiation is considered to play an essential role as a mediator of gene silencing. According to many recent reports, miRNAs have been detected in aqueous humor and are expected to serve as important markers for ocular diseases. Furthermore, exosomes in aqueous humor are considered carriers of miRNAs in intercellular communication within the eye. Therefore, profiling exosomal miRNAs in aqueous humor may be highly beneficial in providing important clues to the pathophysiology of pachychoroid spectrum disease.

Accordingly, as a result of efforts to elucidate the pathogenesis and predict the therapeutic responsiveness of pachychoroid spectrum disease, the present disclosure has been completed.

### [Disclosure]

### [Technical Problem]

An object of the present disclosure is to provide a composition for diagnosis or prognosis prediction of pachychoroid spectrum disease.

Another object of the present disclosure is to provide a method for providing information for diagnosis or prognosis prediction of pachychoroid spectrum disease.

The technical problems of the present disclosure are not limited to the aforementioned problems, and any other technical problems not mentioned herein will be clearly understood from the following description by those skilled in the art.

### [Technical Solution]

To achieve the above objects, an aspect of the present disclosure provides a composition for diagnosis or prognosis prediction of pachychoroid spectrum disease, which comprises an agent for measuring the expression level of miR-184.

Furthermore, the present disclosure provides a method for providing information for diagnosis or prognosis prediction of pachychoroid spectrum disease, which comprises measuring an expression level of miR-184 from a sample obtained from a patient.

### [ Advantageous Effects]

When the composition for diagnosis or prognosis prediction of pachychoroid spectrum disease according to the present disclosure is used, pachychoroid spectrum disease can be accurately diagnosed, and it may serve as a foundation for providing appropriate treatment, thereby being effectively utilized in personalized medicine. Furthermore, when the method for predicting therapeutic responsiveness of pachychoroid spectrum disease is used, the responsiveness to anti-VEGF therapy can be predicted, which may assist in determining an appropriate treatment method.

However, the effects of the present disclosure are not limited to those mentioned above, and other effects not mentioned will be clearly understood by those skilled in the art from the following description.

### [ Description of Drawings]

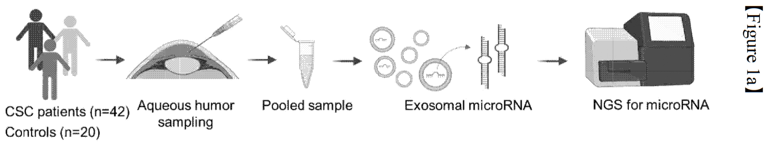

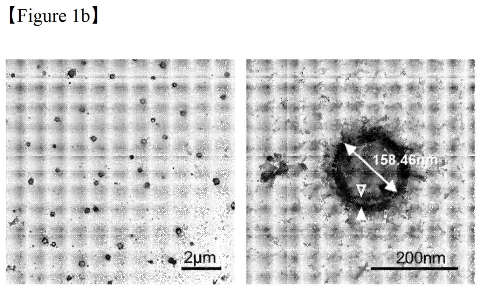

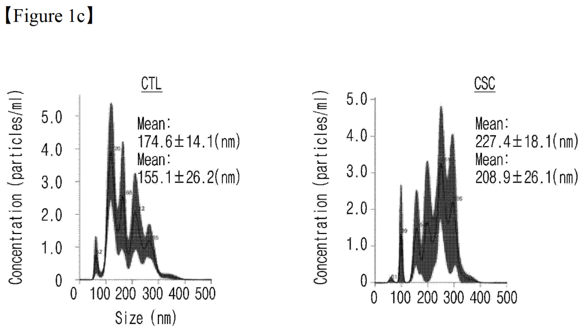

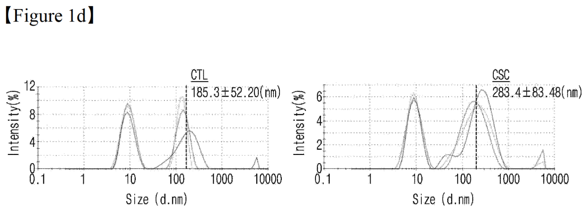

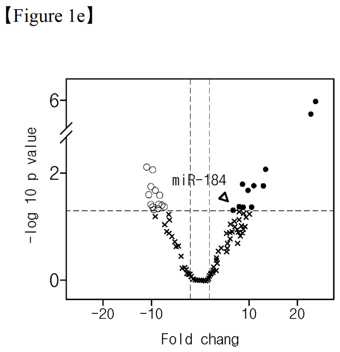

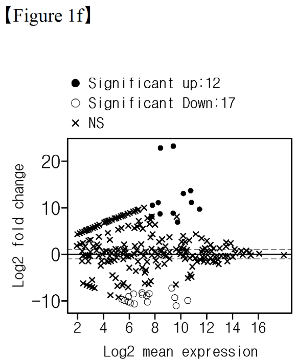

FIG. 1 is a result of exosomal miRNA expression profiles of aqueous humor derived from a control group and patients with central serous chorioretinopathy. FIG. 1A is a schematic view depicting an experimental flow of aqueous humor exosome sequencing. FIG. 1B is a transmission electron microscope image of exosomes in aqueous humor of a single patient. Exosomes were concentrated tenfold after ultracentrifugation. The purified exosomes have a double membrane (inner membrane, indicated by open arrows; outer membrane, indicated by filled arrows), and the size of the exosomes is indicated by double-headed arrows. FIG. 1C is a result of nanoparticle tracking analysis (NTA) of nanosized particles (exosomes) in aqueous humor obtained from a single control and a patient with central serous chorioretinopathy. The patient with central serous chorioretinopathy exhibited a larger average exosome size and a higher exosome concentration than the control group. FIG. 1D is a result of nanoparticle distribution in aqueous humor measured by DLS. The left graph represents the control group (three individuals), and the right graph represents patients with central serous chorioretinopathy (three individuals). The values represent the most intense nanoparticle size ± standard error, and the intense nanoparticle (exosome) sizes distribution is larger in central serous chorioretinopathy than in the control group. FIGS. 1E and 1F are a volcano plot (E) and an MA plot (F), respectively, showing differentially expressed miRNAs between the control group and patients with central serous chorioretinopathy. The dotted lines indicate a log₂ fold change of greater than 2 and a P value of less than 0.05 in FIG. 1E, and a log₂ fold change of greater than 2 in FIG. 1F.

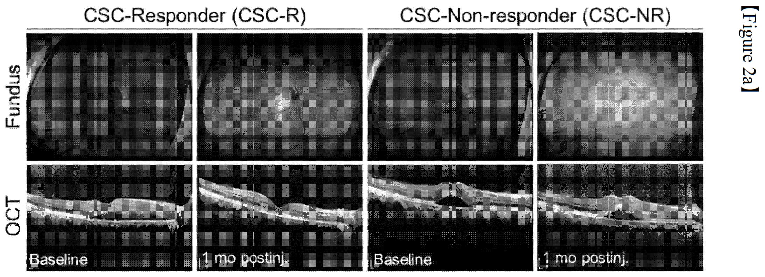

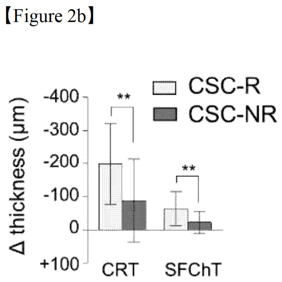

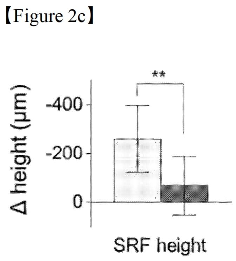

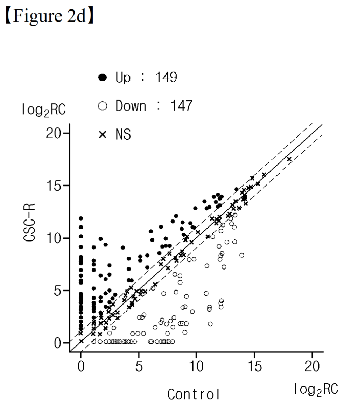

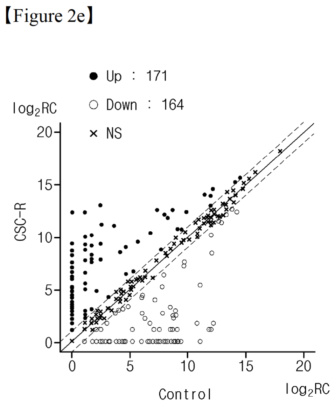

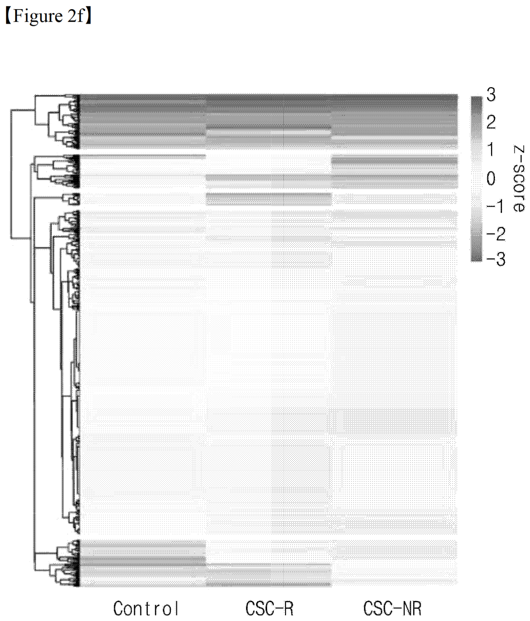

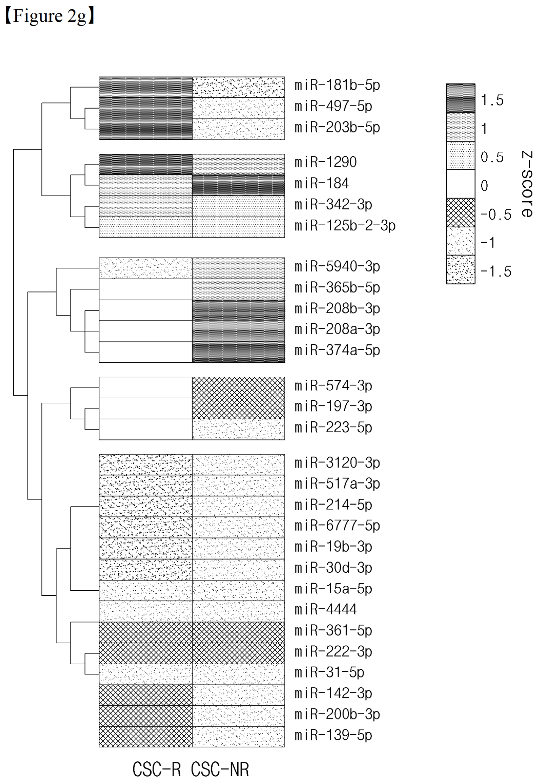

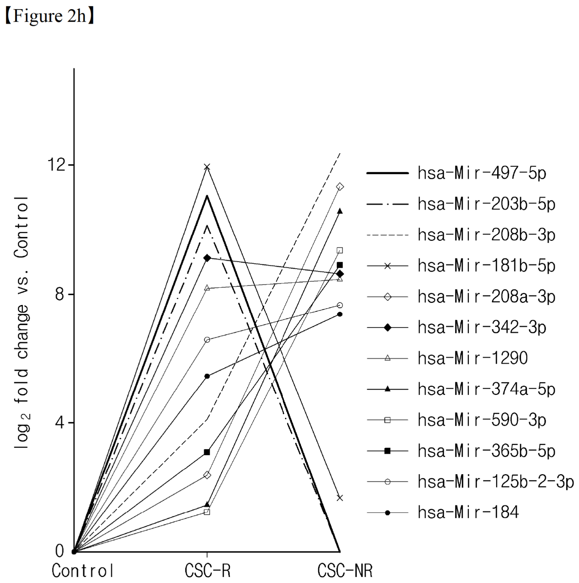

FIG. 2 shows a subgroup analysis of patients with central serous chorioretinopathy according to therapeutic response to intravitreal anti-VEGF treatment. FIG. 2A shows representative wide-field fundus images, autofluorescence images, and optical coherence tomography (OCT) results of patients with central serous chorioretinopathy according to therapeutic response to anti-VEGF treatment. FIGS. 2B and 2C show quantification of changes of OCT parameters (n = 17, responder group [CSC-R]; n = 25, non-responder group [CSC-NR]). FIGS. 2D and 2E are scatter plots showing differences in miRNA expression between the control group and the CSC-R (D) and CSC-NR (E) groups. The red dotted lines indicate a twofold change. FIG. 2F is a heatmap of unsupervised hierarchical clustering. FIG. 2G is a heatmap of differentially expressed miRNAs between the CSC-R and CSC-NR groups. FIG. 2H shows relative miRNA expression in aqueous humor-derived exosomes from the control group, CSC-R, or CSC-NR patients.

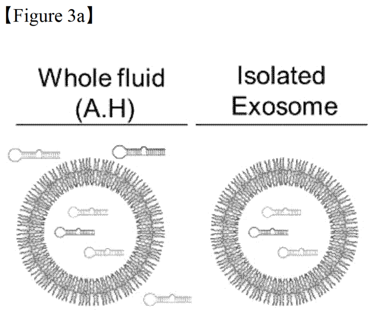

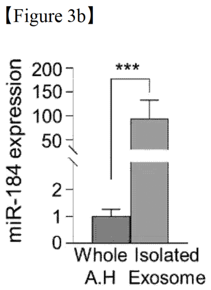

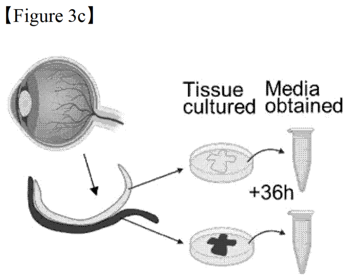

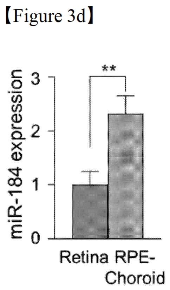

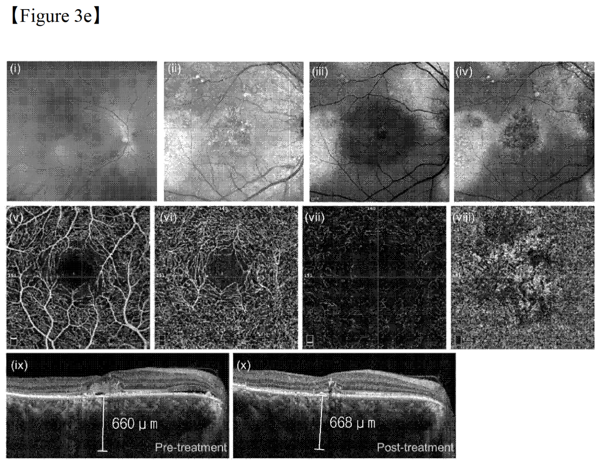

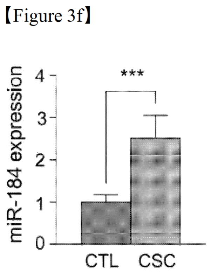

FIG. 3 shows the results of identifying the expression of exosomal miR-184 in aqueous humor from individual patients with central serous chorioretinopathy. FIG. 3A is a schematic view of whole aqueous humor and isolated exosomes, for depicting the location, distribution, and concentration of miRNAs of interest. FIG. 3B shows the results of detecting miR-184 by using probe-based qPCR in aqueous humor of the control group and in exosomes purified from the same aqueous humor samples. The expression of miR-184 isolated from the purified exosomes was significantly higher, indicating that miR-184 is more concentrated in the exosomes. FIG. 3C is a schematic view of tissue culture obtained from donor eyes and culture media for analysis of exosomes secreted from the retina and RPE-choroid of the cultured tissue. FIG. 3D is a result of miR-184 detection using probe-based qPCR in culture media of retinal or RPE-choroid tissue. miR-184 was significantly higher in the RPE-choroid culture medium during the first 36 hours after initiating a new culture. FIG. 3E is a representative multimodal image of a patient with central serous chorioretinopathy. (i) UWF, (ii) SLO-IR, (iii) BAF, (iv) IRAF, (v) OCTA(SCP), (vi) OCTA(DCP), (vii) OCTA(OPL-BRM), (viii) OCTA(choriocapillaris), (ix) baseline OCT, and (x) OCT after one month of anti-VEGF therapy. The choroidal thickness is demarcated in the OCT image. FIG. 3F shows the measurement of the expression of exosomal miR-184 from the aqueous humor of the patient with central serous chorioretinopathy shown in FIG. 3E, which was relatively quantified compared with the control group. Four independently synthesized miRNA cDNAs were amplified in duplicate per cDNA to reduce technical errors. The expression of miR-184 was markedly increased. The graph values are presented as mean ± standard deviation, with *P < 0.05, **P < 0.01, and ***P < 0.001.

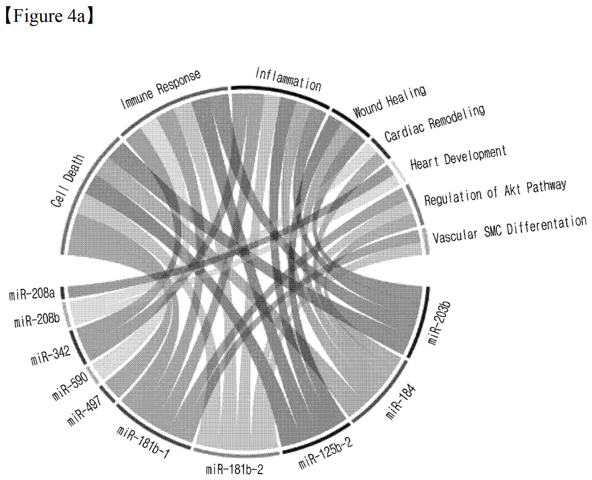

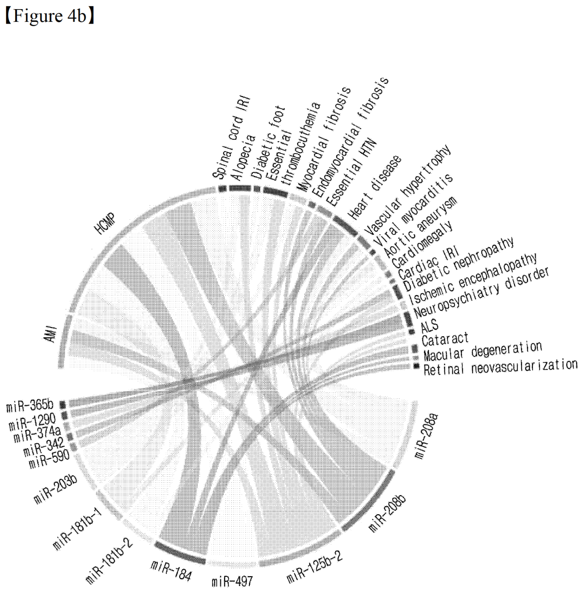

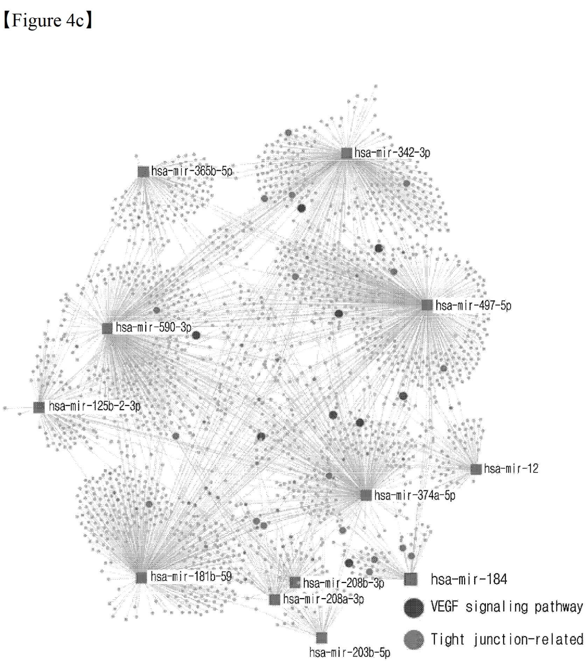

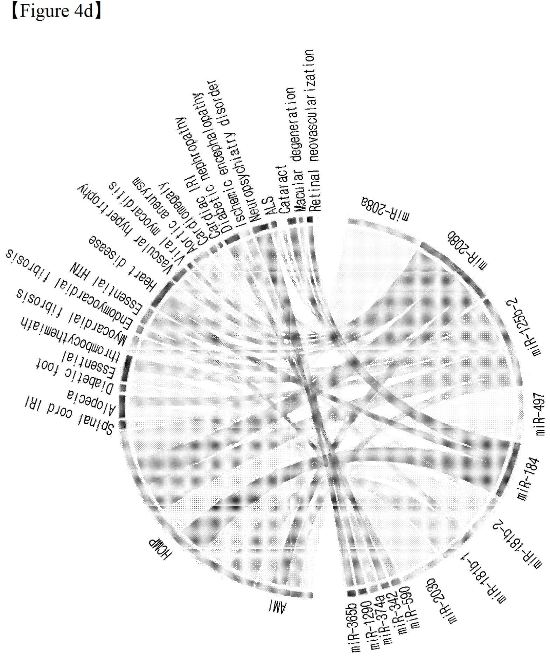

FIG. 4 shows the results of functional and disease association analyses of differentially expressed miRNAs in patients with central serous chorioretinopathy. FIG. 4A is a chord diagram showing connections between differentially expressed miRNAs of enriched biological processes (color-coded). FIG. 4B is a chord diagram showing connections between differentially expressed miRNAs and enriched disease associations (color-coded). FIG. 4C is a graphical representation of the post-transcriptional interaction network between upregulated miRNAs and downregulated target genes. Gene ontology clusters of the VEGF signaling pathway and tight junction-related pathways are indicated in color. FIG. 4D shows the network analysis results of diseases associated with upregulated miRNAs, highlighting age-related macular degeneration (AMD).

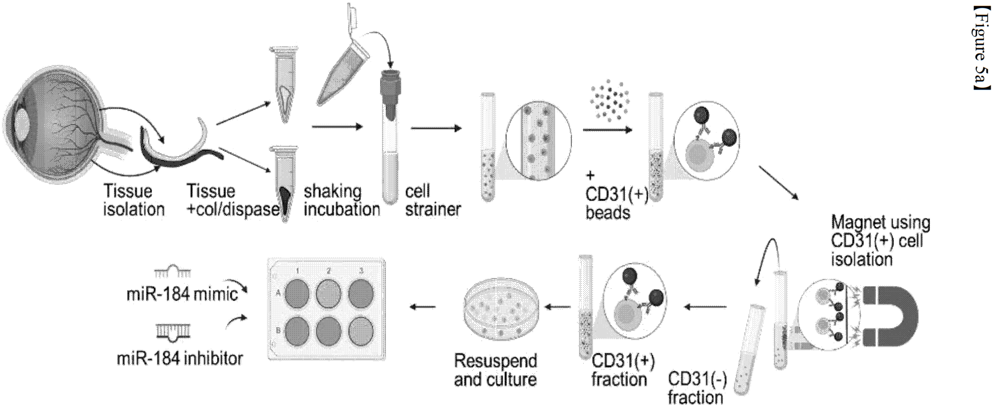

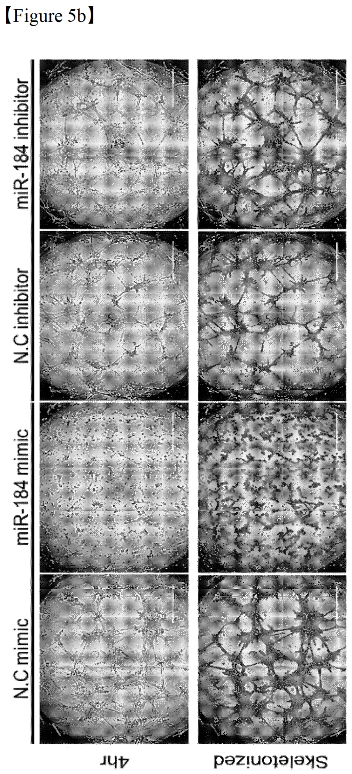

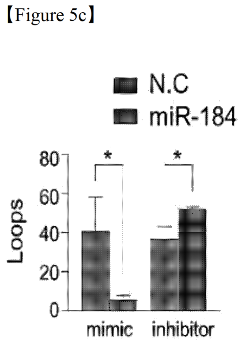

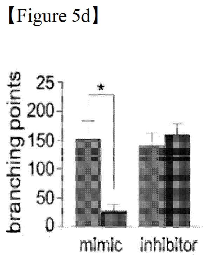

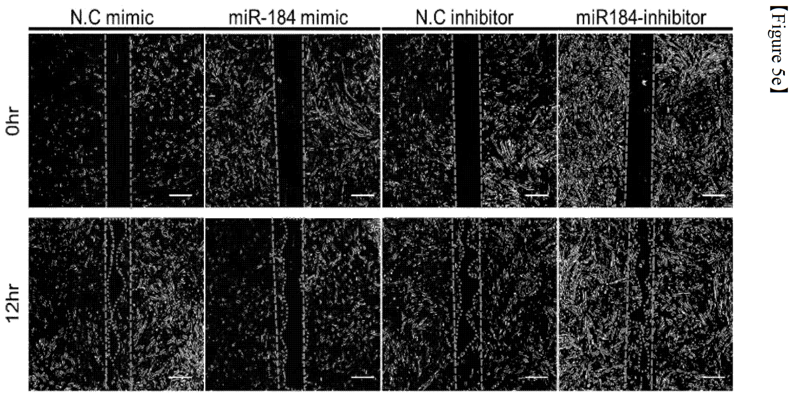

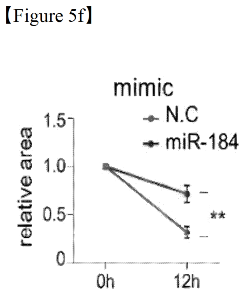

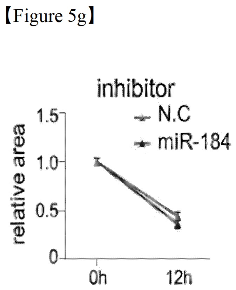

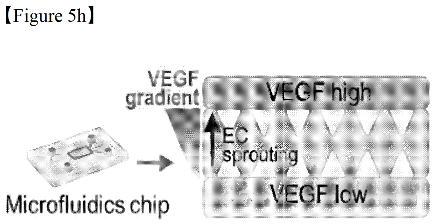

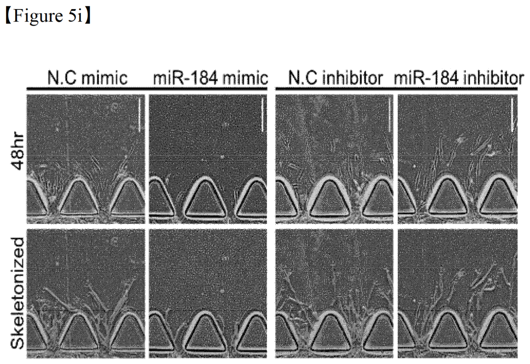

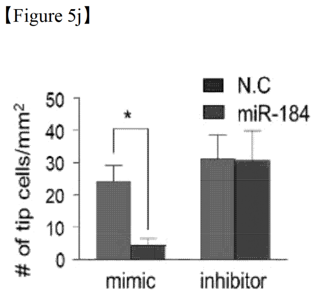

FIG. 5 shows the results identifying that miR-184 inhibits the growth and migration of human primary choroidal endothelial cells (hCECs). FIG. 5A is a schematic view showing the isolation of endothelial cells from donor eyes using CD31(+) magnetic beads and the culture and analysis of cells transfected with a miR-184 mimic and an inhibitor. FIG. 5B shows the results of a 3D tube formation assay using hCECs (primary human choroidal endothelial cells) transfected with a miR-184 mimic or inhibitor, 4 hours after seeding. The images were skeletonized and measured for analysis. FIGs. 5C and 5D respectively show the measurements of total loops and branch points, which are parameters of tube formation, at 4 hours. It was identified that total loops and branch points were markedly decreased in cells transfected with a miR-184 mimic. FIG. 5E shows the results of a scratch wound healing assay using hCECs transfected with a miR-184 mimic or inhibitor. The wound area was measured 2 hours after scratching. FIGs. 5F and 5G show the results of wound area measurements, in which FIG. 5F shows that cells transfected with a miR-184 mimic exhibited reduced wound closure, whereas FIG. 5G shows that cells transfected with a miR-184 inhibitor showed no significant difference. FIG. 5H is a schematic view of an angiogenic sprouting analysis using a microfluidic chip. A 20 ng/ml VEGF gradient was generated to induce endothelial cell sprouting. FIG. 5I shows the results of a sprouting angiogenesis assay using hCECs transfected with a miR-184 mimic or inhibitor. The cells were analyzed 48 hours after the generation of the VEGF gradient. FIG. 5J shows the measurement and analysis of the number of tip cells per millimeter. The miR-184 mimic significantly reduced endothelial sprouting, as evidenced by a decreased number of endothelial tip cells 48 hours after exposure to the VEGF gradient. All analyses were performed in three replicate cultures, and the graph values are presented as mean ± standard deviation. * Statistical significance: *P < 0.05, **P < 0.01; (n = 3); (B, C) scale bar = 1 mm; (E-I) scale bar = 20 µm.

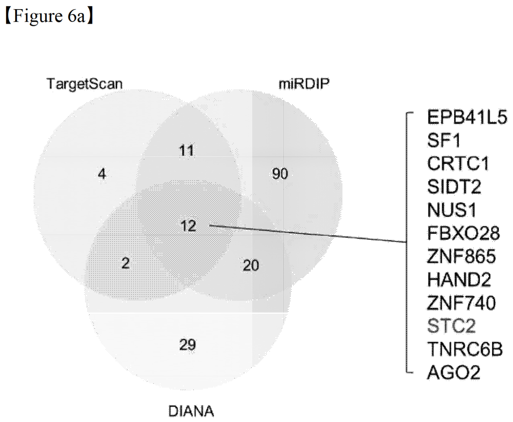

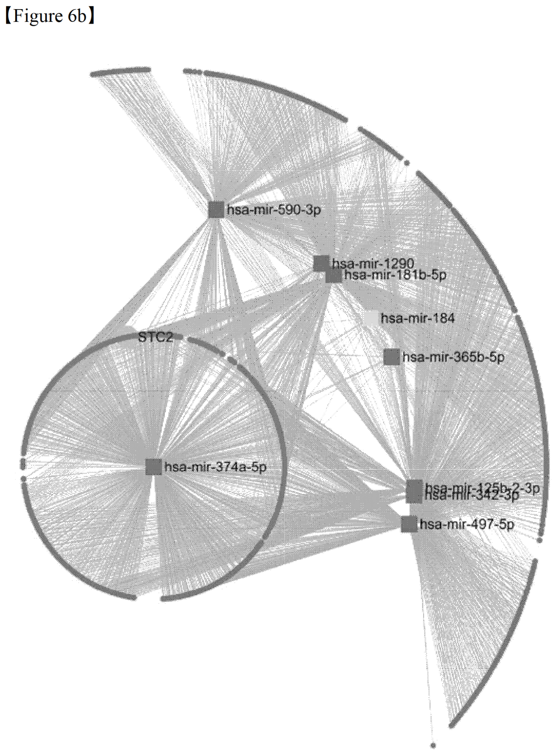

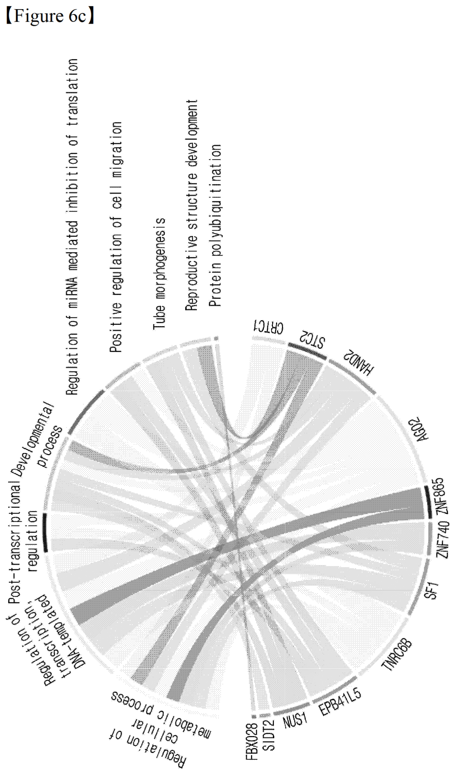

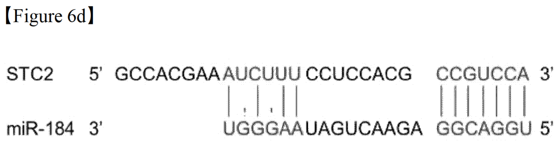

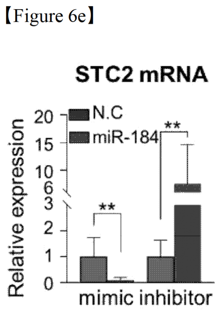

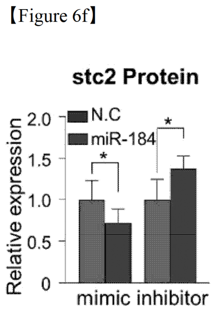

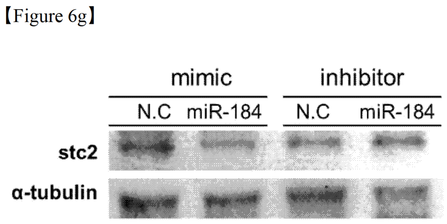

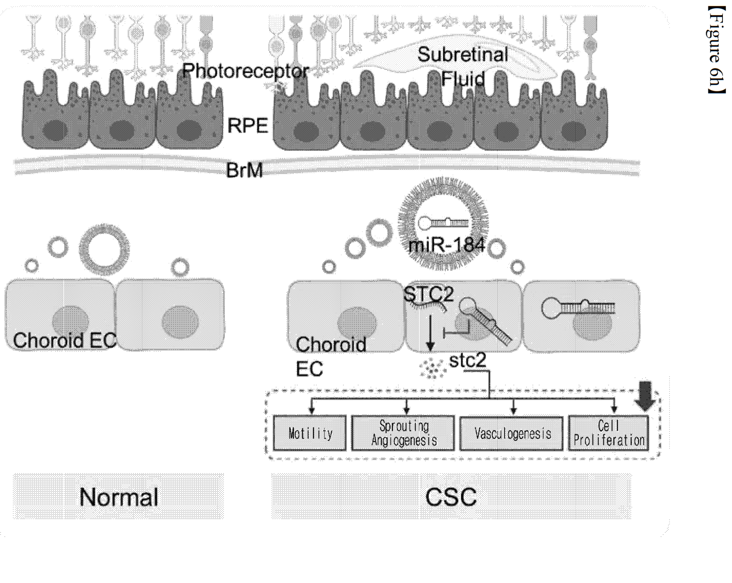

FIG. 6 shows the results identifying STC2 as a potential target gene of miR-184 that regulates angiogenesis, vasculogenesis, motility, and proliferation of choroidal endothelial cells. FIG. 6A is a Venn diagram showing the prediction results of miR-184 target genes from the TargetScan, miRDIP, and DIANA databases. FIG. 6B shows the target gene interaction network of abundant miRNAs isolated from exosomes of the aqueous humor of patients with central serous chorioretinopathy, highlighting STC2 and miR-184. FIG. 6C is a chord diagram (color-coded) showing connections between target genes of miR-184 and biological processes. FIG. 6D shows the predicted miR-184 binding site in the 3'-UTR of human STC2 mRNA. FIG. 6E shows the quantification of STC2 mRNA in hCECs transfected with a miR-184 mimic or inhibitor, 24 hours after transfection. Four independent culture replicates and two amplification replicates were performed. FIGs. 6F and 6G show that the protein level of STC2 in hCECs transfected with a miR-184 mimic or inhibitor was quantified by western blotting 48 hours after transfection. Three independent culture replicates with two SDS-PAGE replicates were performed. FIG. 6H is a schematic view summarizing the function of exosomal miR-184 in choroidal endothelial cells of central serous chorioretinopathy. Exosomes produced from choroidal endothelial cells (ECs) of central serous chorioretinopathy containing miR-184 are delivered to neighboring ECs to inhibit angiogenic pathways by suppressing STC2 transcription in the choroidal ECs, resulting in decreased STC2 protein production and suppression of angiogenic properties. It is indicated by thick red arrows within red dashed boxes.

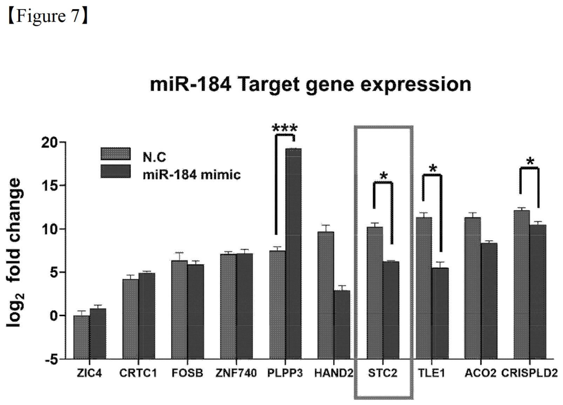

FIG. 7 shows the results verifying that miR-184 target genes were downregulated by expression of miR-184 mRNA in hCECs transfected with a miR-184 mimic. It was identified that STC2 mRNA (indicated by blue boxes) was significantly decreased upon transfection with a miR-184 mimic.

### [ Mode for Invention]

First, terms used in the present disclosure are defined.

As used in the present disclosure, the term "choroidal thickening" generally refers to a condition in which the subfoveal choroidal thickness exceeds 300 µm on swept-source optical coherence tomography (SS-OCT), and the term "pachychoroid spectrum disease" or "pachychoroid spectrum disease group" refers to a condition in which the choroidal thickening causes retinal abnormalities accompanied by changes in the choroidal pigment epithelium. The pachychoroid spectrum disease group is a comprehensive disease group that includes central serous chorioretinopathy (CSC), polypoidal choroidal vasculopathy (PCV), pachychoroid pigment epitheliopathy, and peripapillary pachychoroid. It is known that when the diameter of Haller's layer choroidal vessels increases in pachychoroid spectrum diseases, the relative choroidal pressure rises, which may secondarily cause functional deterioration of the retinal pigment epithelium.

As used in the present disclosure, the term "diagnosis" refers to identifying the presence or characteristics of a pathological condition. The diagnosis in the present disclosure is to identify the presence or occurrence of the pathology of pachychoroid spectrum disease.

As used in the present disclosure, the term "prognosis prediction" refers to anticipating or estimating a medical outcome in advance, and, for the purposes of the present disclosure, it means predicting the course of the disease (progression, improvement, or drug resistance) in patients with pachychoroid spectrum disease. Furthermore, the prognosis includes a positive prognosis (favorable prognosis) or a negative prognosis (unfavorable or poor prognosis), and in the present disclosure, the positive prognosis refers to having therapeutic responsiveness, meaning that the patient is responsive to anti-VEGF therapy, whereas the negative prognosis refers to lacking therapeutic responsiveness to anti-VEGF therapy.

As used in the present disclosure, the term "biomarker" refers to a molecule that is quantitatively or qualitatively associated with the presence of a biological phenomenon, and the biomarker of the present disclosure refers to a substance that may identify the presence of pachychoroid spectrum disease, or a substance that serves as a criterion for predicting whether a patient has a favorable or poor prognosis, and includes non-coding nucleic acids, of which expression levels are increased or decreased in patients with pachychoroid spectrum disease or in patients having therapeutic responsiveness to pachychoroid spectrum disease, compared with a control group.

As used in the present disclosure, the terms "miRNA," "miR," "microRNA," or "micro RNA" refer to 21 to 23 non-coding RNAs that regulate gene expression post-transcriptionally by promoting degradation of target RNAs or inhibiting their translation. The mature sequences of the miRNAs used in the present disclosure may be obtained from the miRNA database (http://www.mirbase.org). In general, microRNA is transcribed as a stem-loop (primary miRNA or pri-miRNA) precursor of a length of about 70-80 nucleotides (nt), which is a hairpin structure called pre-miRNA. Such pri-miRNAs may contain several miRNA precursors and are processed into precursor miRNAs (pre-miRNAs) having hairpin structures by enzymatic action. Subsequently, the pre-miRNA is transported out of the nucleus and its hairpin structure is cleaved in the cytoplasm by the RNase enzyme Dicer. In this process, Dicer binds to the 3' end of the hairpin, cleaves the loop connecting the 3' and 5' arms, and forms an unstable double-stranded miRNA, which ultimately produces mature miR-3p and miR-5p.

Hereinafter, the present disclosure will be described in detail.

### 1. Novel Biomarker

The present disclosure provides miR-184 as a novel biomarker for diagnosis or prognosis prediction of pachychoroid spectrum disease. In an embodiment of the present disclosure, next-generation sequencing (NGS) and bioinformatic analysis were performed to identify specific exosomal miRNAs in aqueous humor samples from patients with pachychoroid spectrum disease and donors. A total of 376 miRNAs were detected by NGS, and compared with the control group, patients with pachychoroid spectrum disease showed 12 significantly upregulated miRNAs and 17 downregulated miRNAs. Among these, miRNAs showing markedly different expression profiles between "CSC-R" (treatment responder) and "CSC-NR" (treatment non-responder) were selected. Among them, miR-184 was upregulated in CSC-R compared with the control group, and was further upregulated in CSC-NR patients compared with CSC-R. It was identified that miR-184 was highly expressed particularly in isolated exosomes compared with the whole aqueous humor, and its statistical significance was also identified.

In an additional embodiment of the present disclosure, the functional role of miR-184 in choroidal endothelial cells was identified by using the miR-184 mimic and inhibitor. Through 3D tube formation analysis, in vitro scratch wound healing analysis, and in vitro 3D microfluidic angiogenesis analysis, it was identified that miR-184 inhibits angiogenesis of hCECs and thereby prevents angiogenic characteristics of CECs in patients with pachychoroid spectrum disease. Accordingly, miR-184 may serve as a potent biomarker for identifying patients with pachychoroid spectrum disease exhibiting high angiogenesis and for predicting responsiveness to anti-VEGF therapy.

Accordingly, in the present disclosure, it was identified that miR-184 may be used for diagnosis or prognosis prediction of pachychoroid spectrum disease, and the prognosis for diagnosis or therapeutic responsiveness may be predicted by using it, and an appropriate treatment direction may be determined according to the predicted prognosis, so that a patient-specific therapeutic method may be provided.

Meanwhile, miR-208b-3p, miR-208a-3p, miR-374a-5p, miR-125b-2-3p, miR-590-3p, miR-365b-5p, miR-497-5p, miR-203b-5p, and miR-181b-5p may also serve as biomarkers for diagnosis or prognosis prediction of pachychoroid spectrum disease.

In a specific embodiment, miRNAs differentially expressed between patients with central serous chorioretinopathy, which is among pachychoroid spectrum diseases, and a normal control group were analyzed, and miRNAs showing differences in expression levels were identified between a group having therapeutic responsiveness to anti-VEGF treatment (treatment responders, CSC-Rs) and a group lacking therapeutic responsiveness (treatment non-responders, CSC-NRs). As a result, as shown in Embodiment 2 and FIGs. 2F to 2H, it was identified that not only miR-184 but also miR-208b-3p, miR-208a-3p, miR-374a-5p, miR-125b-2-3p, miR-590-3p, miR-365b-5p, miR-497-5p, miR-203b-5p, and miR-181b-5p showed changes in expression levels.

Accordingly, it may be seen that, in addition to miR-184, miR-208b-3p, miR-208a-3p, miR-374a-5p, miR-125b-2-3p, miR-590-3p, miR-365b-5p, miR-497-5p, miR-203b-5p, and miR-181b-5p may also be utilized as auxiliary biomarkers for diagnosis or prognosis prediction of pachychoroid spectrum disease.

### 2. Utilization of Novel Biomarkers

### 2-1. Composition for Diagnosis or Prognosis Prediction of Pachychoroid Spectrum Disease

The present disclosure provides a composition for diagnosis or prognosis prediction of pachychoroid spectrum disease, including an agent for measuring an expression level of miR-184.

The agent for measuring the expression level of the miR-184 may be a primer, probe, or antisense nucleotide that specifically binds to the miR-184.

As used in the present disclosure, the term "primer" refers to a short nucleic acid sequence having a free 3' hydroxyl group that may form base pairs with a complementary template and serves as a starting point for replication of the template. In the present disclosure, PCR amplification may be performed by using sense and antisense primers of the marker polynucleotide, and thus, diagnosis or prognosis prediction of pachychoroid spectrum disease may be achieved by determining whether a desired product is generated. The PCR conditions and the lengths of the sense and antisense primers may be modified based on those known in the art.

Furthermore, the primer nucleic acid sequence of the present disclosure may, if necessary, include a label that is directly or indirectly detectable by spectroscopic, photochemical, biochemical, immunochemical, or chemical means. Examples of the label include enzymes (for example, horseradish peroxidase, alkaline phosphatase), radioactive isotopes (for example, ³²P), fluorescent molecules, and chemical groups (for example, biotin).

As used in the present disclosure, the term "probe" refers to a nucleic acid fragment, such as RNA or DNA, ranging from several bases to several hundred bases in length, which may specifically bind to mRNA and is labeled so that the presence or absence of a specific mRNA may be identified. The probe may be prepared in the form of an oligonucleotide probe, a single-stranded DNA probe, a double-stranded DNA probe, or an RNA probe. In the present disclosure, hybridization may be performed by using a probe complementary to the marker miRNA of the present disclosure, and thus, diagnosis or prognosis prediction of pachychoroid spectrum disease may be achieved by determining whether hybridization occurs. Selection of an appropriate probe and hybridization conditions may be modified based on those known in the art.

The primer or probe of the present disclosure may be chemically synthesized by using a phosphoramidite solid-support method or other widely known methods. Such nucleic acid sequences may also be modified by using various means known in the art. Non-limiting examples of such modifications include methylation, capping, substitution with one or more analogs of natural nucleotides, and modifications between nucleotides, for example, modification to uncharged linkages (e.g., methyl phosphonate, phosphotriester, phosphoramidate, carbamate, etc.) or charged linkages (e.g., phosphorothioate, phosphorodithioate, etc.).

The probe and/or primer for detecting the miRNA biomarker according to the present disclosure may be used in various known methods, and various reagents suitable for the specific method used may also be included in the composition according to the present disclosure. Such methods may include, for example, nucleic acid hybridization, polymerization, amplification methods, and hybridization-based ligation, but are not limited thereto.

Nucleic acid hybridization may be performed in a form in which a nucleic acid is bound to a solid support, for example, beads, nanoparticles, or biochip arrays (microarrays), or by using in situ hybridization. The miRNA microarray technology enables the simultaneous analysis of multiple miRNAs. A nucleotide complementary to the miRNA according to the present disclosure may be spotted onto a coated solid support or spotted onto the solid support by an in situ synthesis method. In an embodiment, the miRNA isolated from a biological sample may be detected by incorporation of a label (for example, biotin or a fluorescent dye) that is detected through a hybridization enzymatic reaction with a complementary sequence, for example, a probe, on the solid support. In another embodiment, the miRNA isolated from a biological sample may be labeled with a fluorescent substance, and bind to a corresponding sequence, and the resulting emitted fluorescence signal indicates the presence of a specific miRNA. Microarray fabrication techniques may refer, for example, to Schena et al., 1996, Proc. Natl. Acad. Sci. USA 93(20):10614-10619; Schena et al., 1995, Science 270(5235):467-470; and U.S. Pat. Nos. 5,599,695, 5,556,752, or 5,631,734. In such cases, a detection substance or reagent may be provided in a form bound to a solid support. The detection reagent may be labeled directly for detection or indirectly in a sandwich form, as will be described later.

For measurement of the expression level of the miR-184, nucleic acid polymerization or amplification methods may also be used, which are particularly suitable for detecting miRNAs present in trace amounts. Various known nucleic acid amplification or synthesis methods may be used, including, for example, reverse transcription (RT), reverse transcription polymerase chain reaction (RT-PCR), real-time RT-PCR, polymerase chain reaction (PCR), real-time PCR, quantitative RT-PCR, quantitative PCR, nucleic acid sequence-based amplification (NASBA), ligase chain reaction (LCR), multiple ligatable probe amplification (MLPA), Invader technology (Third Wave), strand displacement amplification (SDA), and transcription-mediated amplification (TMA), but are not limited thereto.

A typical PCR method for amplification of a specific target sequence consists of three operations of denaturation of the template, annealing in which forward and reverse primers bind to the target sequence, and extension by a thermostable polymerase, and these three operations are repeated for multiple cycles, for example, typically 20 or more times. Alternatively, annealing and extension may be performed in the same operation. Because a mature miRNA is single-stranded, a reverse transcription reaction may be performed prior to PCR. The reverse transcription reaction requires the use of a primer and a reverse transcriptase.

In PCR and quantitative PCR, a set of forward and reverse primers or a probe together with the primers may be used. The lengths of the probe and primers are determined by various factors, such as the hybridization temperature, the composition of the target sequence, and the complexity of the target sequence. For example, the probe may have a length of 7 or more nucleotides, and the primers may have a length of about 10 to 35 nucleotides, for example, 15, 20, 25, 30, or 35 nucleotides. The forward primer includes at least one sequence that may specifically bind to the biomarker miRNA and may further include a non-complementary sequence at the 5' end. The sequence of the reverse primer may be independent of the sequence of the biomarker, and multiple miRNA biomarkers may be amplified by using one type of reverse primer, or the reverse primer may include one or more sequences specific to the biomarker.

The amplified product may be analyzed by various methods known in the art, either during the amplification process or after the amplification. Such methods are known in the art and may include, for example, gel electrophoresis, real-time PCR analysis, single strand conformational polymorphism (SSCP), restriction fragment length polymorphism (RFLP), capillary zone electrophoresis (CZE), HPLC-based nucleic acid analyzing technology (WAVE), and microchips, but are not limited thereto.

Furthermore, a ligation technique based on hybridization may be used for quantitative analysis of miRNA. Such methods are known in the art and may include, for example, oligonucleotide ligation (OLA) and a method using HARP-like probes described in U.S. Patent Application Publication No. 2006-0078894, which separates detectable probes bound to a target nucleic acid sequence from unbound probes, but are not limited thereto. Other techniques using ligation may include multiplex ligation-dependent probe amplification (MLPA) (Schouten et al., Nucleic Acids Research 30:e57 (2002)). The technique is based on ligation that occurs only when a pair of probes bind adjacent to each other on a target sequence, and the ligated probes include primer binding sites so that they may be amplified by PCR.

In the hybridization, amplification, and/or hybridization-based ligation reactions as described above, a hybridized or amplified miRNA product may be detected through staining or labeling of the target, or staining or labeling of the primer or probe. Detection may be performed by using techniques known in the art, and those skilled in the art may select an appropriate method in consideration of the sensitivity of detection and/or the amount of the target. Depending on the sensitivity of the detection method and/or the amount of the target, amplification prior to detection may not be necessary.

The label for detection is not limited thereto, but includes compounds that may generate or quench detectable fluorescent, chemiluminescent, or bioluminescent signals, such as light emission, light scattering, or light absorption substances, and reference may be made to Garman A., NonRadioactive Labeling, Academic Press, 1997. The fluorescent substance is not limited thereto, but may include any fluorescent moiety that may generate a detectable signal, for example, fluorescein (e.g., U.S. Patent No. 6,020,481), rhodamine (e.g., U.S. Patent No. 6,191,278), benzophenoxazine (e.g., U.S. Patent No. 6,140,500), energy transfer fluorescent dyes including a donor and an acceptor (e.g., U.S. Patent No. 5,945,526 and Korean Patent Publication No. 10-2016-0022017), cyanine (e.g., WO 1997-45539), lissamine, phycoerythrin, Cy2, Cy3, Cy3.5, Cy5, Cy5.5, Cy7, FluorX (Amersham), Alexa 350, Alexa 430, AMCA, BODIPY 630/650, BODIPY 650/665, BODIPY-FL, BODIPY-R6G, BODIPY-TMR, BODIPY-TRX, Cascade Blue, 6-FAM, Fluorescein Isothiocyanate, HEX, 6-JOE, Oregon Green 488, Oregon Green 500, Oregon Green 514, Pacific Blue, REG, Rhodamine Green, Rhodamine Red, Renographin, ROX, SYPRO, TAMRA, Tetramethylrhodamine, and/or Texas Red.

Furthermore, the label may include an intercalator, a minor groove binder, or a cross-linkable functional group, which is a compound that may enhance, stabilize, or affect the binding of nucleic acids, for example, ethidium bromide and SYBR Green, but is not limited thereto (see Blackburn et al., eds.). Reference may be made to DNA and RNA Structure in Nucleic Acids in Chemistry and Biology (1996). Accordingly, the composition according to the present disclosure may include a reagent used in at least one of the above-described methods.

The overlapping description is the same as that described above in "1. Novel Biomarker," and thus the description will be omitted. The biomarker according to the present disclosure described above may be detected through quantitative and/or qualitative analysis at levels of the presence or absence of miRNA, an expression amount thereof, a change in the expression amount, or a difference in the expression amount, and may be used for diagnosis or prognosis prediction of pachychoroid spectrum disease.

In one embodiment, the prognosis prediction may be to predict therapeutic responsiveness, and the therapeutic responsiveness may be to predict responsiveness to anti-VEGF therapy. In the present disclosure, the expression "having therapeutic responsiveness" means that, when anti-VEGF therapy is performed on a patient with pachychoroid spectrum disease, subretinal fluid is completely absorbed in SD-OCT, indicating that the treatment is effective, and the expressions "having no therapeutic responsiveness" or "treatment non-responsiveness" mean that, although anti-VEGF therapy is performed, subretinal fluid remains even after one month, indicating that the treatment is ineffective.

In one embodiment, the pachychoroid spectrum disease may be central serous chorioretinopathy, polypoidal choroidal vasculopathy, pachychoroid pigment epitheliopathy, and peripapillary pachychoroid.

In the present disclosure, the term "central serous chorioretinopathy" refers to a disease, in which fluid accumulates in the posterior pole of the retina centered on the macula, which is the central part of the retina, causing partial detachment of the macular region. Furthermore, "polypoidal choroidal vasculopathy (PCV)" is characterized by the presence of an abnormal vascular network in inner choroidal vessels and vascular aneurysms that are expanded into polyp-like structures at the ends thereof. "Pachychoroid pigment epitheliopathy" refers to a condition in which persistent congestion occurs in the choroidal vasculature, causing pressure damage to the capillaries, and continuous leakage of fluid toward Bruch's membrane and the retinal pigment epithelium occurs, resulting in damage to the pigment epithelium. "Peripapillary pachychoroid" refers to a type of disease characterized by a thickened choroid and the presence of intraretinal or subretinal fluid around the optic disk, and exhibits features, such as retinal pigment epithelium detachment, choroidal hyperpermeability, and dilated pachyvessels. Preferably, the pachychoroid spectrum disease of the present disclosure may be central serous chorioretinopathy.

In an embodiment, the composition may further include at least one selected from the group consisting of an agent for measuring an expression level of miR-208b-3p, an agent for measuring an expression level of miR-208a-3p, an agent for measuring an expression level of miR-374a-5p, an agent for measuring an expression level of miR-125b-2-3p, an agent for measuring an expression level of miR-590-3p, an agent for measuring an expression level of miR-365b-5p, an agent for measuring an expression level of miR-497-5p, an agent for measuring an expression level of miR-203b-5p, and an agent for measuring an expression level of miR-181b-5p.

Here, as described above, the agent for measuring the expression level is the same as the "agent for measuring an expression level of miR-184," and thus the description thereof will be omitted.

### 2-2. Kit

The present disclosure provides a kit for diagnosis or prognosis prediction of pachychoroid spectrum disease, which includes a composition for diagnosis or prognosis prediction of the pachychoroid spectrum disease.

Reagents that may be included in the kit and detection using the same may refer to the above-mentioned descriptions. In an aspect of the present disclosure, the kit is used for nucleic acid amplification, and particularly, for amplification using RT-PCR. In this case, the kit may include a primer set and/or a probe required for the RT-PCR reaction, a buffer, a reverse transcriptase, and Taq polymerase. In another aspect, the kit may further include a nuclease that may remove single-stranded nucleic acids. Various buffers known in the art may be used, for example, a Tris-HCl buffer at pH 9.0 may be used, but the present disclosure is not limited thereto. The reverse transcriptase and Taq polymerase are commercially available, and for example, a polymerase capable of a hot start reaction, such as AmpliTaq Gold (Applied Biosystems, USA), may be used, and an appropriate concentration, for example, 1.5 mM to 2.5 mM of MgCl₂, may be included.

The kit according to the present disclosure further includes a positive control group, a negative control group, and instructions for use. The negative control group may be a sample that does not contain miRNA, and the positive control group may include one or more of the target miRNAs to be detected.

In the specification, the kit of the present disclosure described above may additionally include various polynucleotide molecules, enzymes, various buffers, and reagents. An optimal amount of a reagent used in a specific reaction may be easily determined by those skilled in the art having acquired the disclosures of the present specification.

The kit may further include an instruction for performing the method of the present disclosure, and the instruction may describe that when the expression level of miR-184 is two times or more higher than that of a normal control group, it is diagnosed as pachychoroid spectrum disease. Furthermore, the instruction may describe that when the expression level of miR-184 is at least two times higher than that of a normal control group and at least two times lower than that of a non-response group, it is determined that there is therapeutic responsiveness to pachychoroid spectrum disease. Additionally, the instruction may describe that, when measuring the expression levels of miR-208b-3p, miR-208a-3p, miR-374a-5p, miR-125b-2-3p, miR-590-3p, miR-365b-5p, miR-497-5p, miR-203b-5p, and miR-181b-5p, and it is determined that there is anti-VEGF therapeutic responsiveness to pachychoroid spectrum disease, i) when the expression levels of miR-208b-3p, miR-208a-3p, miR-374a-5p, miR-125b-2-3p, miR-590-3p, and miR-365b-5p are at least two times higher than those of a normal control group and at least two times lower than those of a non-response group, or ii) when the expression levels of miR-497-5p, miR-203b-5p, and miR-181b-5p are at least two times higher than those of both a normal control group and a non-response group.

The descriptions of the normal control group, the treatment non-response group, and the therapeutic responsiveness are the same as those described later in "2-3. Method for Providing Information for Diagnosis or Prognosis Prediction of Pachychoroid Spectrum Disease," and thus the description thereof will be omitted.

### 2-3. Method for Providing Information for Diagnosis or Prognosis Prediction of Pachychoroid Spectrum Disease

The present disclosure provides a method for providing information for diagnosis or prognosis prediction of pachychoroid spectrum disease, which includes an operation of measuring an expression level of miR-184 from a sample collected from a patient.

In the present disclosure, the term "patient" refers to a subject having, or expected to have, pachychoroid spectrum disease, and may include, without limitation, humans who need to determine whether to receive anti-VEGF therapy, primates, such as chimpanzees, pets such as dogs and cats, livestock animals, such as cattle, horses, sheep, and goats, rodents, such as mice and rats, and aquaculture fish.

In an embodiment, the "sample" used for analysis includes a biological sample that may identify a specific biomarker that may diagnose or predict the prognosis of pachychoroid spectrum disease, which may be distinguished from a normal condition, such as blood, plasma, serum, saliva, tears, or aqueous humor. Preferably, the liquid sample isolated from the eye, tears, or aqueous humor may be used, and most preferably, the aqueous humor or exosomes derived from the aqueous humor may be used.

In the present disclosure, the term "aqueous humor" refers to a transparent fluid located in the space between the iris and the cornea, which functions to maintain intraocular pressure and the rounded shape of the front of the eye, to supply nutrients to the inner surface, and to remove waste products from the inner front portion of the eye. In the specification, the terms "aqueous humor," "aqueous fluid," "aqueous," and "AH" may be used interchangeably.

In a specific embodiment, exosomes were isolated from the aqueous humor to identify the expression of miR-184, and the expression in the whole aqueous humor was compared with the expression in the isolated exosomes (Embodiment 3 and FIG. 3). As a result of detecting miR-184 by using probe-based qPCR, it was identified that the expression level of miR-184 was significantly higher in the isolated exosomes compared to the whole aqueous humor fluid (FIGS. 3A and 3B).

The expression level of miR-184 may be measured by various methods, including reverse transcription reaction, reverse transcription polymerase chain reaction (RT-PCR), real-time RT-PCR, polymerase chain reaction (PCR), real-time PCR, quantitative RT-PCR, quantitative PCR, nucleic acid sequence-based amplification (NASBA), ligase chain reaction (LCR), multiple ligatable probe amplification (MLPA), Invader technology (Third Wave), strand displacement amplification (SDA), and transcription mediated amplification (TMA), but is not limited thereto, and any known method for measuring miRNA may be used without limitation.

Specific means (methods) and detection reagents for implementing the method according to the present disclosure may refer to the descriptions provided above.

In an embodiment, the method may further include an operation of measuring expression levels of miR-208b-3p, miR-208a-3p, miR-374a-5p, miR-125b-2-3p, miR-590-3p, miR-365b-5p, miR-497-5p, miR-203b-5p, and miR-181b-5p from a sample obtained from the patient.

In an embodiment, the method for providing information for diagnosis or prognosis prediction of pachychoroid spectrum disease may further include an operation of determining that the patinet has pachychoroid spectrum disease when the measured expression level of miR-184 is higher than that of a normal control group.

In another embodiment, the method for providing information for diagnosis or prognosis prediction of pachychoroid spectrum disease may further include an operation of determining that the patient has pachychoroid spectrum disease when the measured expression level of miR-184 is higher than that of a normal control group, and the measured expression level of at least one of miR-208b-3p, miR-208a-3p, miR-374a-5p, miR-125b-2-3p, miR-590-3p, miR-365b-5p, miR-497-5p, miR-203b-5p, and miR-181b-5p is higher than that of the normal control group.

The "normal control group" may refer to healthy individuals who do not have pachychoroid spectrum disease and may include a group of patients who have undergone cataract surgery but have not been diagnosed with diabetes or retinal disease.

The expression "the expression level is higher than that of a normal control group" may refer to cases in which the level is at least 1.3-fold, 1.5-fold, 1.8-fold, 2-fold, 4-fold, 8-fold, 16-fold, or 32-fold higher than that of a normal individual, and may particularly be 4-fold or higher.

In a specific embodiment, miRNAs differentially expressed between patients with pachychoroid spectrum disease and a normal control group were analyzed, and miRNAs showing differences in expression levels were identified. As a result, as shown in Example 2 and FIGS. 2F to 2H, it was identified that not only miR-184 but also miR-208b-3p, miR-208a-3p, miR-374a-5p, miR-125b-2-3p, miR-590-3p, and miR-365b-5p showed changes in expression levels.

In one embodiment, the prognosis prediction may be to predict therapeutic responsiveness, and the therapeutic responsiveness may be to predict responsiveness to anti-VEGF therapy.

Specifically, in patients with pachychoroid spectrum disease, anti-VEGF therapy is used to reduce the hyperpermeability of choroidal endothelial cells. Well-known anti-VEGF agents, such as bevacizumab, ranibizumab, and aflibercept, may be used for the treatment of patients with pachychoroid spectrum disease by lowering the level of subretinal fluid. However, while there are patients who show good therapeutic responsiveness to anti-VEGF treatment, there also exists a group of patients who exhibit suboptimal or secondary responses. Accordingly, separating patients with pachychoroid spectrum disease to determine whether to proceed with anti-VEGF therapy when establishing a treatment strategy is very useful, as it allows for the determination of an appropriate treatment direction and enables the performance of only clinically necessary treatments.

Here, the method for providing information for diagnosis or prognosis prediction of pachychoroid spectrum disease may additionally include an operation of determining that patient has therapeutic responsiveness to therapy for pachychoroid spectrum disease when the measured expression level of miR-184 is higher than that of a normal control group and lower than that of a non-response group.

The "normal control group" may refer to healthy individuals who do not have pachychoroid spectrum disease and may include a group of patients who have undergone cataract surgery but have not been diagnosed with diabetes or retinal disease. The "non-response group" refers to patients in whom subretinal fluid (SRF) remains even after one month despite undergoing anti-VEGF treatment. In the present disclosure, the "non-response group" includes "treatment non-responders" and may be used interchangeably with "CSC non-responders" or "CSC-NRs" in specific embodiments and figures. In addition, the expressions "having no therapeutic responsiveness" or "treatment non-responsiveness" mean that, although anti-VEGF treatment is performed, subretinal fluid (SRF) remains even after one month, indicating that the treatment is ineffective. In contrast, the "treatment response group" refers to patients in whom subretinal fluid (SRF) is completely absorbed in SD-OCT after undergoing anti-VEGF treatment. In the present disclosure, the "treatment response group" in specific embodiments and figures includes "treatment responders" and may be used interchangeably with "CSC responders" or "CSC-Rs." Furthermore, the expression "having therapeutic responsiveness" means that, when anti-VEGF treatment is performed on a patient with pachychoroid spectrum disease, subretinal fluid (SRF) is completely absorbed in SD-OCT, indicating that the treatment is effective.

The expression "the expression level is lower than that of the non-response group" may refer to cases in which the level is at least 1.3-fold, 1.5-fold, 1.8-fold, 2-fold, 4-fold, 8-fold, 16-fold, 32-fold, 64-fold, 128-fold, or 256-fold lower than that of the non-response group, and may particularly be 4-fold or lower.

Accordingly, by obtaining and comparing the expression level or expression pattern of miRNA from samples obtained from the normal control group and the non-response group, and from a patient whose prognosis of pachychoroid spectrum disease is to be determined, the prognosis of the patient may be accurately predicted.

In a specific embodiment, to gain insight into the differences in miRNA expression in response to anti-VEGF treatment, exosomal miRNA expression patterns were compared between treatment responders and non-responders (see Example 2 and FIG. 2). The results for differentially expressed miRNAs showed that the expression level of miR-184 (hsa-miR-184) was one of the miRNAs increased in both treatment responders and non-responders compared to the control group (FIGS. 2D to 2G). Furthermore, it was identified that the expression level of miR-184 was one of the miRNAs significantly increased in treatment non-responders compared to treatment responders (FIGS. 2F to 2H).

Furthermore, when the measured expression level of miR-184 is higher than that of a normal control group and lower than that of a non-response group, and at the same time satisfies at least one of the following conditions i) and ii), it may be determined that there is therapeutic responsiveness to therapy for pachychoroid spectrum disease.

i) the measured expression level of at least one selected from the group consisting of miR-208b-3p, miR-208a-3p, miR-374a-5p, miR-125b-2-3p, miR-590-3p, and miR-365b-5p is higher than that of the normal control group and lower than that of the non-response group,

ii) the measured expression level of at least one selected from the group consisting of miR-497-5p, miR-203b-5p, and miR-181b-5p is higher than those of the normal control group and the non-response group.

Based on the results of the method for providing information for diagnosis or prognosis prediction of pachychoroid spectrum disease of the present disclosure, a treatment decision for pachychoroid spectrum disease may be made. After being determined as belonging to the treatment response group, anti-VEGF therapy may be appropriately applied to the patient alone or in combination as a treatment method for pachychoroid spectrum disease.

Hereinafter, the present disclosure will be described in detail with reference to embodiments.

However, the following embodiments are merely illustrative of the present disclosure, and the scope of the present disclosure is not limited to these embodiments.

### [Preparation Example]

### Patients

In the embodiments of the present disclosure, a study was conducted on 42 eyes of 42 patients diagnosed with CSC at the Department of Ophthalmology, Yeungnam University College of Medicine, Daegu, Republic of Korea. Among patients who had undergone cataract surgery, 20 eyes from 20 patients who had not been diagnosed with diabetes or retinal disease were selected as the control group, and for exosome size analysis and individual miR-184 measurement, 15 eyes from 15 patients with CSC and 22 eyes from 22 patients with cataracts as controls were enrolled at Asan Medical Center.. The diagnosis of CSC was defined as the presence of a fluorescein leakage pattern in fluorescein angiography (FA) and a clear accumulation of subretinal fluid in spectral domain optical coherence tomography (SD-OCT), and among the patients diagnosed with CSC, those with a symptom duration between six weeks and four months were selected as subacute CSC patients. Among these patients, those who had choroidal neovascularization, a history of CSC treatment, comorbid ophthalmic diseases induced along with CSC, a history of diabetes, a history of intraocular surgery, a history of systemic or topical use of a carbonic anhydrase inhibitor within one month, or a history of intravitreal steroid injection were excluded. The study protocol was approved by the Institutional Review Board (Yeungnam University IRB No. 2019-10-056-002 and Asan Medical Center IRB No. 2020-1945-0002) and was conducted in accordance with the principles of the Declaration of Helsinki.

### Multi-Image Analysis of Patients

All patients underwent bilateral ophthalmic examinations, including slit-lamp examination and fundus examination, and were imaged using ultra wide-field fundus photography (UWF; Optos California, Optos plc, UK), ultra wide-field autofluorescence (UWF-AF), ultra wide-field fluorescein angiography (UWF-FA), scanning laser ophthalmoscope infrared imaging (SLO-IR), blue autofluorescence (BAF), infrared autofluorescence (IRAF), and spectral domain optical coherence tomography (SD-OCT; Spectralis, Heidelberg Engineering, Heidelberg, Germany). Furthermore, choroidal images were obtained using the enhanced depth imaging (EDI) technique in spectral domain optical coherence tomography (SD-OCT). To exclude choroidal neovascularization, ultra wide-field indocyanine green angiography (UWF-ICGA) or optical coherence tomography angiography (OCTA) was performed as needed. The central retinal thickness (CRT) was automatically measured within the central 1 mm area from the fovea by using SD-OCT segmentation analysis. The subfoveal choroidal thickness (SFChT), subretinal fluid (SRF) height, and pigment epithelial detachment (PED) height were manually measured on SD-OCT images using the virtual calipers of the software (Heidelberg Eye Explorer ver. 6.0) (FIG. 1A).

### Patient Subgroup Classification

All CSC patients were treated with intravitreal bevacizumab (IVB). CSC patients were classified into a treatment response group and a non-response group according to follow-up examination results. CSC responders (CSC-Rs) were defined as patients in whom subretinal fluid was completely absorbed on SD-OCT one month after IVB injection. CSC non-responders (CSC-NRs) were defined as patients in whom subretinal fluid remained on SD-OCT one month after IVB injection.

### Aqueous Humor Sampling

Before analysis, patients were informed of and signed written consent forms for sample collection and scientific use, and aqueous humor samples were collected before IVB injection in subacute CSC patients and during cataract surgery in the control group. Aqueous humor samples collected from CSC patients were classified as CSC responders and CSC non-responders according to their response to bevacizumab. Each collected sample group was pooled into protein low-bind tubes. The pooled volume of each group was 3.5 mL for the control group (N=20), 2.0 mL for CSC responders (N=17), and 3.0 mL for CSC non-responders (N=25).

### Exosomal miRNA Sequencing

Exosomal RNA was isolated using the ExoLutE^{®} exosome isolation kit (Rosetta, Seoul, Korea) according to the manufacturer's instructions. RNA quality control and quantification were performed using an RNA 6000 Pico Chip (Agilent Technologies, Amstelveen, The Netherlands) on an Agilent 2100 Bioanalyzer, and RNA quantification was carried out using a NanoDrop 2000 spectrophotometer system (Thermo Fisher Scientific, Waltham, MA, USA).

For RNA from the control and experimental groups, library construction was performed using the NEBNext Multiplex Small RNA Library Prep Kit (New England BioLabs, Inc., USA) according to the manufacturer's instructions. For library construction, 1 µg of total RNA from each sample was used for adapter ligation, followed by cDNA synthesis using reverse transcriptase with adapter-specific primers. PCR was performed for library amplification, and the libraries were purified using the QIAquick PCR Purification Kit (Qiagen, Inc., Germany) and AMPure XP beads (Beckman Coulter, Inc., USA). The yield and size distribution of the small RNA libraries were evaluated by using a high-sensitivity DNA analysis on an Agilent 2100 Bioanalyzer instrument (Agilent Technologies, Inc., USA). High-throughput sequencing was performed on a NextSeq 500 system (Illumina, San Diego, CA, USA) using a single-end 75 sequencing method. The raw data set was obtained from the sequence read archive (SRA) database of the NCBI.

### Data Analysis

Sequence reads were mapped using the Bowtie2 software tool to obtain binary alignment map (BAM) files. Mature miRNA sequences were used as references for mapping. Read counts mapped to mature miRNA sequences were extracted from the alignment files by using Bioconductor and bedtools (v2.25.0) in the R statistical programming language (version 3.2.2; R Development Core Team, 2011).

To determine the expression levels of miRNAs, read counts were used, the quantile normalization method was applied for comparison among samples, and differential expression gene (DEG) analysis was performed by using DESeq2. Significant DEGs were defined as those with P < 0.05 and an absolute log₂ fold change greater than 2. miRNet and miRWalk 2.0 were utilized for miRNA target and functional studies. The graphical representations were generated by using ggplot2.

### [Embodiment 1]

### Exosomal miRNA Analysis in the Aqueous Humor of CSC Patients

In this embodiment, 42 eyes from 42 patients diagnosed with CSC and 20 eyes from 20 patients who had undergone cataract surgery without being diagnosed with diabetes or retinal disease were used as the control group (FIG. 1A). CSC eyes were obtained from 34 male and 8 female patients (50.8 ± 8.6 years old), and control group eyes were obtained from 5 male and 15 female patients (62.2 ± 3.6 years old). Baseline characteristics and baseline OCT analysis are presented in [Table 1] below.

**[Table 1]**

| | Control group (n=20) | CSC (n=42) | p-value |
|---|---|---|---|
| Sex (Male : Female) | 5 : 15 | 34 : 8 | < 0.001 * |
| Age | 62.2 ± 3.6 | 50.8 ± 8.6 | < 0.001 † |
| Baseline SFChT (µm) | 233.92 ± 95.24 | 372.08 ± 104.60 | < 0.001 † |

For analysis, aqueous humor samples were collected and pooled (3.0 mL) (FIG. 1A). Aqueous humor samples were obtained with individually sealed sterile syringes placed on frozen ice. All samples were prepared within 4 hours after collection, and the frozen aqueous humor samples were thawed at 4 °C for 2 hours. The frozen aqueous humor samples were syringe-filtered using 4 mm RC membrane syringe filters (Corning, #431212, USA) to minimize sample loss and were collected into protein low-bind tubes (Protein LoBind Tube; Eppendorf, #022431081, Germany). The filtered aqueous humor was centrifuged at 15,000 × g for 30 minutes at 4 °C to remove cellular debris and proteins, and the supernatant was then collected for exosome purification and analysis. Before profiling exosomal miRNA expression, exosomes were first purified and identified from the patients' aqueous humor by using transmission electron microscopy (TEM), and subsequently analyzed by using nanoparticle tracking analysis (NTA) and dynamic light scattering (DLS) (FIG. 1B).

### Transmission Electron Microscopy (TEM) of Exosomes

One hundred microliters of freshly filtered and collected aqueous humor samples were ultracentrifuged at 120,000 × g for 2 hours at 4 °C. The supernatant was carefully discarded, and the pellet was resuspended in 10 µL of sterile PBS. The resuspended liquid was dropped onto a nickel-carbon grid (Electron Microscopy Sciences, #CF200-Ni-50, USA), and for fixation, 2.5% glutaraldehyde was dropped onto the grid at a 1:1 volume ratio for 10 minutes. A 1% uranyl acetate solution was dropped onto the grid to allow gentle flow, and the grid was left at room temperature (RT) for 2 minutes for exosome staining. The grid was gently washed three times by dropping sterile distilled water and then dried. Images were obtained using a transmission electron microscope (H-7000; Hitachi, Japan).

### Exosome Purification

One hundred microliters of filtered and collected aqueous humor samples were transferred to new tubes, and ExoQuick-TC Ultra for cell culture media (System Biosciences, #EQULTRA-20TC-1, USA) was used. Exosomes were isolated by modifying the manufacturer's protocol for a smaller sample volume. The buffer was used at one-twentieth of the volume recommended in the protocol. The isolated exosomes were ultracentrifuged at 120,000 × g for 2 hours at 4 °C, and the supernatant was carefully discarded without disturbing the pellet. The pellet was resuspended or lysed in the buffer for subsequent analyses.

### Exosome Size Analysis

To analyze the size of the purified exosomes, the pellet was resuspended in sterile DEPC-treated aqueous 1× phosphate-buffered saline (PBS) to a final volume of 100 µL. Aqueous humor or purified and resuspended exosome samples were diluted 1:3 with sterile PBS. For dynamic light scattering (DLS) analysis, particle size measurement was performed by using a Zetasizer (Nano-ZS ZEN3600, Malvern Panalytical, UK). The analysis results were obtained after performing 10 measurements per sample for three different samples from the disease group. The concentration was corrected by using a dilution factor of 3.00e+0. For nanoparticle tracking analysis (NTA), NTA was performed on individual samples by using a NanoSight (NS300, Malvern Panalytical, UK). The results were recorded five times for 60 seconds per sample at 25 °C. The concentration was corrected by using a dilution factor of 3.00e+0.

### Exosomal miRNA Isolation and Analysis

For the purified exosome pellets, 35 µL of QIAzol was added for miRNA isolation, and the miRNeasy Micro Kit (Qiagen, #1071023, Germany) was used according to the provided protocol. Reverse transcription was performed with 10.0 ng of RNA by using a miR-184-specific reverse transcription primer, and quantification of miR-184 was conducted by using probe-based real-time qPCR with the TaqMan MicroRNA Reverse Transcription Kit (Applied Biosystems, #4366596, USA), the miR-184-specific TaqMan MicroRNA Assay (Applied Biosystems, #4427975, USA), and the TaqMan Universal Master Mix II (Applied Biosystems, #4440040, USA). Real-time qPCR was performed by using a Bio-Rad CFX Connect system and analyzed with CFX Maestro software.

As a result, the average size and concentration of exosomes increased in CSC patients compared to the control group (FIGS. 1C and 1D). As a result of exosomal miRNA profiling, a total of 376 miRNAs were consistently detected across all groups by NGS. Furthermore, compared to the control group, CSC patients showed 12 significantly upregulated miRNAs and 17 downregulated miRNAs (FIGS. 1E and 1F). Accordingly, after analyzing differentially expressed miRNAs by fold change and volcano plot filtering, the present inventors identified that miR-184 was significantly upregulated in CSC compared to the control group (FIG. 1E).

### [Embodiment 2]

### Subgroup Analysis According to Anti-VEGF Treatment Response

To investigate differences in miRNA expression according to anti-VEGF therapeutic responsiveness, CSC-R (treatment responders) and CSC-NR (treatment non-responders) patients were compared (FIG. 2). Of the ocular samples obtained from 42 CSC patients, 17 eyes were classified as CSC-Rs, and 25 eyes were classified as CSC-NRs (FIG. 2A). There was no significant difference in baseline OCT findings between the two groups (Table 2).

**[Table 2]**

| | CSC Treatment Responders (CSC-Rs) (n=17, 40.5%) | CSC Treatment Non-Responders (CSC-NRs) (n=25, 59.5%) | p-value |
|---|---|---|---|
| **Baseline OCT Results** | | | |
| Baseline CRT (µm) | 424.65 ± 129.56 | 458.52 ± 122.41 | 0.395 † |
| Baseline SFChT (µm) | 379.86 ± 118.08 | 366.82 ± 96.58 | 0.697 † |
| Baseline SRF height (µm) | 256.25 ± 164.81 | 225.94 ± 126.07 | 0.504 † |
| Baseline PED Height (µm) | 63.18 ± 38.18 | 61.51 ± 96.04 | 0.938 † |

| **Changes in OCT Results One Month After Bevacizumab Injection** | | | |
|---|---|---|---|
| ΔCRT (µm) | -198.73 ± 122.32 | -88.00 ± 125.43 | 0.010 † |
| ΔSFChT (µm) | -63.92 ± 51.81 | -23.00 ± 33.24 | 0.005 † |
| ΔSRF Height (µm) | -259.73 ± 138.00 | -67.18 ± 121.72 | <0.001 † |
| Δ PED Height (µm) | -28.94 ± 41.48 | -11.69 ± 30.88 | 0.146 † |

In the table, CSC denotes central serous chorioretinopathy; CRT denotes central retinal thickness; SFChT denotes subfoveal choroidal thickness; SRF denotes subretinal fluid; and PED denotes pigment epithelial detachment. The average values of CRT, SFChT, and SRF height in the CSC-R group significantly decreased compared with those in the CSC-NR group one month after IVB injection (FIGS. 2B and 2C). FIGS. 2D to 2H show the exosomal miRNA expression patterns of CSC patients according to treatment response. The results of differentially expressed miRNAs showed that miR-184 (hsa-miR-184) was one of the upregulated miRNAs in both CSC-R and CSC-NR patients compared to the control group (FIGS. 2D to 2G). Furthermore, miR-184 was one of the miRNAs that were further upregulated in CSC-NR patients compared to CSC-R patients (FIGS. 2F to 2H). Accordingly, the present inventors focused on elucidating the role of miR-184 in CSC patients.

### [Embodiment 3]

### Identification of Exosomal miR-184 Expression in Aqueous Humor of CSC Patients

To identify exosomal miR-184 expression in CSC patients, exosomes were isolated from aqueous humor, and the expression in the entire aqueous humor fluid was compared with that in the isolated exosomes (FIG. 3A).

Aqueous humor samples or exosome-purified solutions purified from the same aqueous humor samples were syringe-filtered by using a 4 mm RA membrane syringe filter, and the filtered aqueous humor was collected. TRIzol LS (Invitrogen, #10296028, USA) was added to the filtered aqueous humor according to the manufacturer's protocol. The separation of miRNA and the detection of miR-184 were performed by using the exosomal miRNA analysis procedure described above. As a result of detecting miR-184 by using probe-based qPCR, miR-184 expression was found to be significantly upregulated (approximately 100-fold increase) in the isolated exosomes compared to the whole aqueous humor, indicating that the concentrated miR-184 resides inside the exosomes rather than in the aqueous humor fluid (FIG. 3B). Additionally, to investigate the origin of exosomal miR-184 in the eye, retinal and RPE-choroid complex tissue cultures were performed by using donor eyes (FIG. 3C).

### Primary Retinal and RPE-Choroid Tissue Culture from Human Donor Eyes

Primary retinal and RPE-choroid tissues were isolated from the eyes of a 60-year-old female donor with no history of metabolic or ocular disease. Retinal and choroid tissues were isolated from the donor eyes immediately after corneal closure and were radially cut into four sections. The tissues were cultured in 60 pi culture dishes in a mixture of culture media for ocular tissue culture: serum-free DMEM/F-12 supplemented with Exo-free FBS (System Biosciences, #EXO-FBS-250A-1, USA), Neurobasal medium supplemented with B27, Pericyte Growth Medium, and Endothelial Growth Medium were mixed at appropriate ratios for the retinal and RPE-choroid tissues, respectively, and the cultures were maintained for 36 hours.

### Analysis of Exosomal miRNA Derived from Primary Human Tissues

The culture media were collected 36 hours after tissue incubation. Exosomes from the collected media and from the fresh control media were purified using ExoQuick-TC Ultra (System Biosciences, #EQULTRA-20TC-1, USA) for tissue culture media, following the manufacturer's protocol used for aqueous humor exosome purification, and were separated as pellets by ultracentrifugation.

### Isolation and Culture of Primary Human Choroidal Endothelial Cells

Primary human choroidal endothelial cells (hCECs) were isolated from the eyes of a 33-year-old female donor and a 31-year-old male donor with no history of metabolic or ocular disease. Choroidal tissues were isolated from donor eyes immediately after corneal closure and dissociated into single cells by using 1 U of Collagenase/Dispase (Roche, #10269638001, Germany) in a shaking incubator (37 °C, 200 rpm) for 2 hours, followed by filtration through a 40 µm cell strainer. CD31-positive endothelial cells were isolated by using the Dynabeads magnetic cell separation system with anti-CD31 Dynabeads (ThermoFisher, #11155D, USA) according to the manufacturer's protocol. CD31-positive hCECs were cultured up to passage 2 on 1% gelatin-coated dishes with Microvascular Endothelial Cell Growth Medium-2 (EGM2-MV; LONZA, #CC-3202, Switzerland) under conditions of 37 °C and 5% CO₂.

After 36 hours of tissue culture, exosomal miR-184 expression was more than twofold higher in exosomes from the RPE-choroid culture medium than in those from the retinal culture medium, indicating that exosomal miR-184 originated predominantly from the RPE-choroid rather than from the retina (FIG. 3D).

To identify the expression of exosomal miR-184 in individual patients, qPCR analysis of miR-184 was performed on aqueous humor-derived exosomes from patients with CSC and from the control group. CSC patients who reported decreased visual acuity for six months were selected based on multimodal imaging (FIG. 3E). (i) UWF, (ii) SLO-IR, (iii) BAF, (iv) IRAF, (v) OCTA(SCP), (vi) OCTA(DCP), (vii) OCTA(OPL-BRM), (viii) OCTA(CC), (ix) baseline OCT, and (x) OCT after one month of anti-VEGF therapy. The choroidal thickness is distinguished in the OCT image. Exosomal miR-184 expression from the aqueous humor of CSC patients was measured and quantitatively compared with that of the control group. Compared with the miRNA sequencing results, CSC patients showed more than a twofold increase in miR-184 expression levels relative to the control group. It could be identified that miR-184 expression was elevated in CSC patients who exhibited suboptimal responses to anti-VEGF therapy (FIG. 3F).

### [Embodiment 4]

### Functional and Disease Association Analysis of Differentially Expressed miRNAs in CSC Patients

To investigate the functional effects of the miRNAs, gene ontology analysis of biological processes was performed. FIG. 4A is a chord diagram showing the connections between differentially expressed miRNAs and enriched biological processes, distinguished by color, and FIG. 4B is a chord diagram showing the connections between differentially expressed miRNAs and enriched disease associations, also distinguished by color. All upregulated miRNAs associated with functional pathways were identified using an FDR cutoff value of 0.05.

As a result, the present inventors identified that miR-184 was significantly associated with the regulation of cell apoptosis, inflammation, wound healing, cardiac remodeling, and the Akt pathway. To explore the physiological effects of the differentially expressed miRNAs, those that were potentially associated with cardiovascular and CNS-related disorders were further evaluated (FIG. 4B). In particular, retinal dysfunctions such as macular degeneration and retinal neovascularization were identified to be associated with miR-184 (FIGs. 4B and 4D). Furthermore, it was identified from the gene network analysis that the upregulated miRNAs in CSC patients, including miR-184, were significantly associated with the VEGF signaling pathway and the tight junction pathway, both of which are closely related to the regulation of vascular permeability (FIG. 4C).

Accordingly, the investigation focused on miR-184, which could be considered a contributing factor to the pathophysiology of CSC development and to suboptimal responses to anti-VEGF therapy.

### [Embodiment 5]

### Identification of Functional Role of miR-184

To interpret the significance of miR-184 elevation in CSC patients, the present inventors performed in vitro analyses by using miR-184 transfection in primary cultured human choroidal endothelial cells (hCECs) to verify the functional role of miR-184 in choroidal endothelial cells (FIG. 5A).

### miRNA mimic/inhibitor transfection

To verify the functional role of miR-184, a miR-184 mimic, inhibitor, and non-targeting negative control RNA (Bioneer Co. Ltd., Daejeon, Korea) were used. The sequences of the miR-184 mimic and inhibitor are as follows.

**[Table 3]**

| Name | Sequence (5'→3') | Sequence ID No. |
|---|---|---|
| hsa-miR-184-mimic | UGG ACG GAG AAC UGA UAA GGG U | 1 |
| hsa-miR-184-inhibitor | UGG ACG GAG AAC UGA UAA GGG U | 2 |

Cells were transfected with a miR-184 mimic, inhibitor, or negative control RNA by using Lipofectamine^{™} RNAiMAX Transfection Reagent (Invitrogen, #13778150) according to the manufacturer's instructions. The synthetic miRNA oligonucleotides were complexed with the transfection reagent in reduced-serum Opti-MEM medium and then added to the cells. Cells were used for subsequent analyses 24 to 48 hours after transfection.

### 3D Tube Formation Analysis

hCECs transfected with the miR-184 mimic/inhibitor or the negative control were seeded onto 96-well plates coated with Matrigel^{®} Matrix (Corning, NY, USA) at a density of 2×10⁴ cells per well. Cells were incubated at 37 °C with 5% CO₂ for 4 hours, and images were taken at 2 and 4 hours after seeding. Tube formation parameters were analyzed by using the WimTube online software (Onimagin, Córdoba, Spain).

As a result, the miR-184 mimic significantly reduced tube formation of hCECs, as evidenced by the decreased numbers of loops and branching points in endothelial cells, whereas the miR-184 inhibitor significantly increased the total number of loops (FIGs. 5B to 5D).

### In Vitro Scratch Wound Healing Analysis

Furthermore, to determine how miR-184 affects cell migration, an in vitro scratch wound healing analysis was performed.

hCECs transfected with the miR-184 mimic/inhibitor or the negative control were seeded onto 24-well plates at a density of 2×10⁵ cells per well and reached confluence within 24 hours. The cell monolayer was scratched using a 200 µL pipette tip and then incubated at 37 °C with 5% CO₂. Because the doubling time of endothelial cells exceeds 17 hours, cell images were captured immediately after scratching and at 12 hours to analyze wound closure resulting from cell migration. The closed wound area was quantified and normalized to the initially created wound area.

As a result, the scratch wound healing analysis of hCECs revealed that miR-184 mimic significantly inhibited endothelial cell migration, whereas the miR-184 inhibitor had no significant effect on migration (FIGs. 5E to 5G).

### In Vitro 3D Microfluidic Angiogenesis Analysis

The present inventors further performed a microfluidic chip analysis to evaluate the effect of miR-184 on angiogenic sprouting in response to VEGF (FIG. 5H).

The microfluidic plastic chip and the chip holder for maintaining chip humidity were purchased from AIM Biotech (AIM Biotech, Singapore). A collagen type I solution (2 mg/mL; Corning, NY, USA) was gently pipetted into the gel-filling inlets of the device and polymerized for 30 minutes at 37 °C with 5% CO₂ in a humidified chip holder. A 1/10 diluted human plasma fibronectin solution (Sigma-Aldrich, #F0895, USA) was injected into the microchannels, and the device was incubated in a humidified chip holder at 37 °C with 5% CO₂ for 1 hour. hCECs transfected with the miR-184 mimic/inhibitor or the negative control were seeded into one of the fluidic channels at a density of 6×10⁴ cells per channel. At 24 hours after seeding, recombinant human VEGF165 (Peprotech, #100-20, Rocky Hill, NJ, USA) was added to the growth medium at final concentrations of 40 and 20 ng/mL according to the manufacturer's instructions, generating a VEGF gradient as previously demonstrated by the present inventors in prior studies. The cells were incubated at 37 °C with 5% CO₂, and the VEGF-containing medium was replaced every 24 hours. Cell sprouting was monitored and imaged every 24 hours. The number of tip cells per millimeter was counted from the acquired images for the sprouting angiogenesis parameters. Interestingly, the miR-184 mimic significantly reduced endothelial sprouting, as evidenced by the decreased number of endothelial tip cells observed 48 hours after exposure to the VEGF gradient (FIGs. 5I and 5J).

In conclusion, these in vitro results identified that miR-184 suppresses angiogenesis in hCECs, suggesting that it may serve as a mechanism to prevent the angiogenic characteristics of CECs in CSC patients.

### [Embodiment 6]

### Target Gene Analysis of miR-184

To identify the targets of miR-184 in the pathophysiology of CSC, the present inventors predicted target genes through a Venn diagram analysis (FIG. 6A). As a result, 12 common target genes were identified across the TargetScan, miRDIP, and DIANA databases.

Meanwhile, it was identified that the target genes of miR-184 were downregulated by miR-184 mRNA expression in hCECs transfected with the miR-184 mimic. As illustrated in FIG. 7, STC2 was identified as the most strongly suppressed angiogenesis-related gene, excluding transcription factors, in hCECs treated with the miR-184 mimic, and because network analysis of the miRNA indicated a strong connection between STC2 and miR-184 (FIG. 6B), STC2 was evaluated as a strong candidate target gene of miR-184.

To predict the functional effects of the common target genes of miR-184 including STC2, a gene ontology analysis of biological processes was performed (FIG. 6C). Interestingly, STC2 was significantly associated with cell development and metabolic regulation. Accordingly, through bioinformatic prediction, a miR-184 binding site in the 3'-UTR of human STC2 mRNA was identified (FIG. 6D).

Furthermore, to identify whether miR-184 transcriptionally affects STC2 expression, the level of STC2 mRNA in hCECs was measured after transfection and inhibition experiments using a miR-184 mimic and inhibitor. Interestingly, the miR-184 mimic significantly downregulated the level of STC2 mRNA, and the miR-184 inhibitor significantly upregulated the level of STC2 mRNA (FIGS. 6E and 6F). As illustrated in FIG. 6H, it was identified that exosomes produced from choroidal endothelial cells (ECs) of central serous chorioretinopathy containing miR-184 are delivered to neighboring ECs to inhibit angiogenic pathways by suppressing STC2 transcription in the choroidal ECs, resulting in decreased STC2 protein production and suppression of angiogenic properties.

### STC2 Western Blot Analysis

Furthermore, after the transfection and inhibition experiments, the protein level of STC2 in hCECs was measured. Cells transfected with the miR-184 mimic/inhibitor or a negative control group for 48 hours were washed three times with PBS. After removing PBS, the cells were immediately frozen in liquid N₂. For cell lysis, cold 1× Pierce RIPA buffer (Thermo Scientific, #89900, US) containing 1× HALT phosphatase inhibitor cocktail (Thermo Scientific, #78420, US) and 1× HALT phosphatase inhibitor cocktail (Thermo Scientific, #78430, US) was immediately added to the cell lysate, the cells were scraped from the plastic surface, and collected into protein low-bind tubes. After incubation on ice for 10 minutes, the cell lysate was centrifuged at 13,000 × g for 30 minutes at 4 °C, and the supernatant containing proteins was collected. The protein concentration was measured using a Pierce BCA assay kit (Thermo Scientific, #23227, US) according to the manufacturer's protocol. After electrophoresis, the samples were blotted onto an NC membrane (GE Healthcare Life Science, #10600114, Germany). The blotted membrane was blocked using EveryBlot Blocking Buffer (Bio-Rad, #12010020, US). The antibodies used for detection were rabbit anti-STC2 (Abcam, #ab63057, US), mouse anti-α-tubulin (Santa Cruz, #SC-5286, US), goat anti-rabbit HRP (Genetex, #GTX213110-01, US), and goat anti-mouse HRP (Genetex, #GTX213111-01, US), all diluted to the concentrations recommended by the manufacturers.

As a result, the protein level of STC2 followed the trend observed in the mRNA results (FIG. 6G), and accordingly, it was identified that miR-184 suppresses STC2 expression in hCECs. Overall, these results indicate that miR-184 may affect several cellular processes related to angiogenesis, vasculogenesis, endothelial cell motility, and cell proliferation by targeting STC2 in CECs (FIG. 6H). miR-184 may be compensatorily upregulated in CSC patients with highly angiogenic properties of CECs. In summary, it was identified that miR-184 may serve as a potent biomarker for identifying CSC patients with high angiogenic activity and for predicting suboptimal responses to anti-VEGF therapy in CSC patients.

### Statistical Analysis

The representative values were expressed as the mean ± standard deviation (SD). Statistical significance was analyzed by using Welch's t-test and Student's t-test, and represented as *P < 0.05, **P < 0.01, and ***P < 0.001. Statistical analyses were performed by using R x64 version 4.1.1.

### Image Analysis

Microscopic images for cellular functional analysis were imaged by using an inverted microscope (Olympus IX70, Japan) with DP Controller software. The images were analyzed and quantified, by using ImageJ (v.1.52p; National Institutes of Health (NIH), Bethesda, MD, USA), a Java-based imaging software available in the public domain (http://rsb.info.nih.gov/ij).

The representative embodiments of the present disclosure have been described above by way of example, but the scope of the present disclosure is not limited to the specific embodiments described above, and those skilled in the art will be able to make appropriate modifications within the scope set forth in the claims of the present application.

## Claims

1. A composition for diagnosis or prognosis prediction of pachychoroid spectrum disease, the composition comprising an agent for measuring an expression level of miR-184.

2. The composition of claim 1, wherein the agent for measuring the expression level of miR-184 is at least one selected from the group consisting of a primer, a probe, and an antisense nucleotide that specifically binds to the miR-184.

3. The composition of claim 1, further comprising at least one selected from the group consisting of an agent for measuring an expression level of miR-208b-3p, an agent for measuring an expression level of miR-208a-3p, an agent for measuring an expression level of miR-374a-5p, an agent for measuring an expression level of miR-125b-2-3p, an agent for measuring an expression level of miR-590-3p, an agent for measuring an expression level of miR-365b-5p, an agent for measuring an expression level of miR-497-5p, an agent for measuring an expression level of miR-203b-5p, and an agent for measuring an expression level of miR-181b-5p.

4. The composition of claim 1, wherein the prognosis prediction is prediction of therapeutic responsiveness.

5. The composition of claim 4, wherein the therapeutic responsiveness is responsiveness to anti-VEGF therapy.

6. The composition of claim 1, wherein the pachychoroid spectrum disease is at least one selected from the group consisting of central serous chorioretinopathy, polypoidal choroidal vasculopathy, pachychoroid pigment epitheliopathy, and peripapillary pachychoroid.

7. A kit for diagnosis or prognosis prediction of pachychoroid spectrum disease, the kit comprising the composition of any one of claims 1 to 6.

8. A method for providing information for diagnosis or prognosis prediction of pachychoroid spectrum disease, the method comprising measuring an expression level of miR-184 from a sample obtained from a patient.

9. The method of claim 8, wherein the sample is at least one selected from the group consisting of blood, plasma, serum, saliva, tears, and aqueous humor.

10. The method of claim 9, wherein the sample is aqueous humor.

11. The method of claim 10, wherein the sample is an exosome derived from aqueous humor.

12. The method of claim 8, further comprising:
measuring an expression level of at least one selected from the group consisting of miR-208b-3p, miR-208a-3p, miR-374a-5p, miR-125b-2-3p, miR-590-3p, miR-365b-5p, miR-497-5p, miR-203b-5p, and miR-181b-5p from the sample obtained from the patient.

13. The method of claim 8, further comprising:
determining that the patient has pachychoroid spectrum disease if the measured expression level of miR-184 is higher than that of a normal control group.

14. The method of claim 12, further comprising:
determining that the patient has pachychoroid spectrum disease if the measured expression level of miR-184 is higher than that of a normal control group and the measured expression level of at least one selected from the group consisting of miR-208b-3p, miR-208a-3p, miR-374a-5p, miR-125b-2-3p, miR-590-3p, miR-365b-5p, miR-497-5p, miR-203b-5p, and miR-181b-5p is higher than that of the normal control group.

15. The method of any one of claims 8 to 12, wherein the prognosis prediction is prediction of therapeutic responsiveness.

16. The method of claim 15, wherein the therapeutic responsiveness is responsiveness to anti-VEGF therapy.

17. The method of claim 16, further comprising:
determining that the patient has therapeutic responsiveness to therapy for pachychoroid spectrum disease if the measured expression level of miR-184 is higher than that of a normal control group and lower than that of a non-response group; or
determining that the patient has therapeutic responsiveness to therapy for pachychoroid spectrum disease if the measured expression level of miR-184 is higher than that of the normal control group and lower than that of the non-response group and, at the same time, satisfies at least one of the following conditions i) and ii):
i) the measured expression level of at least one selected from the group consisting of miR-208b-3p, miR-208a-3p, miR-374a-5p, miR-125b-2-3p, miR-590-3p, and miR-365b-5p is higher than that of the normal control group and lower than that of the non-response group; and
ii) the measured expression level of at least one selected from the group consisting of miR-497-5p, miR-203b-5p, and miR-181b-5p is higher than those of the normal control group and the non-response group.

18. The method of claim 8, wherein the pachychoroid spectrum disease is at least one selected from the group consisting of central serous chorioretinopathy, polypoidal choroidal vasculopathy, pachychoroid pigment epitheliopathy, and peripapillary pachychoroid.
